# EUROPEAN PATENT APPLICATION

(11) **EP 4 722 246 A1**
(43) Date of publication of application: **08.04.2026**
(21) Application number: 24814539.3
(22) Date of filing: 30.05.2024
(51) Int. Cl.: C07K 16/28, A61K 39/395, A61P 35/00

(54) **TEM8-TARGETED ANTIBODY OR ANTIGEN-BINDING FRAGMENT THEREOF, AND USE THEREOF**

(30) Priority: 31.05.2023 CN 202310636114
(71) Applicant: Inxmed (Nanjing) Co., Ltd., Nanjing, Jiangsu 210000 (CN)
(72) Inventor: LU, Shiqiang, Nanjing, Jiangsu 210000 (CN); LIU, Xuebin, Nanjing, Jiangsu 210000 (CN); MA, Yue, Nanjing, Jiangsu 210000 (CN); LU, Yuhong, Nanjing, Jiangsu 210000 (CN); PANG, Ran, Nanjing, Jiangsu 210000 (CN); WANG, Zaiqi, Nanjing, Jiangsu 210000 (CN)
(74) Representative: Vossius & Partner Patentanwälte Rechtsanwälte mbB
(86) International application number: PCT/CN2024/096348
(87) International publication number: WO 2024/245342

(57) **Abstract**

The present invention belongs to the field of antibodies, and specifically relates to a TEM8 binding molecule, especially to an antibody that specifically recognizes TEM8 and a fragment thereof. Furthermore, the present invention also relates to a nucleic acid or host cell comprising the antibody or the fragment thereof, a drug comprising the antibody or the fragment thereof, and a treatment and diagnostic method using the antibody and the fragment thereof, or the use thereof.

## Description

The present application is based on and claims priority to Chinese Patent Application No. 202310636114.4, filed on May 31, 2023, which is incorporated herein by reference in its entirety.

### TECHNICAL FIELD

The present invention belongs to the field of antibodies, and specifically relates to a TEM8 binding molecule, especially to an antibody that specifically recognizes TEM8 and a fragment thereof. Furthermore, the present invention also relates to a nucleic acid or host cell comprising the antibody or the fragment thereof, a drug comprising the antibody or the fragment thereof, and a treatment and diagnostic method using the antibody and the fragment thereof, or the use thereof.

### BACKGROUND OF THE INVENTION

Angiogenesis is a process of developing and forming new blood vessels from existing capillaries or postcapillary veins, which is important for various physiological processes, including organ growth, embryonic development, and wound healing. Angiogenesis also plays an important part in many diseases, especially in tumorigenesis and tumor growth, where tumors require an adequate blood supply to sustain their growth. By comparing the vascular endothelial cell gene expression profiles in normal tissues and tumor tissues, 46 genes were found to be highly expressed in tumor vascular endothelial cells and thus named as Tumor Endothelial Marker (TEM) molecules, in which TEM8 especially has better tumor specificity, as described in Croix *et al.* (Croix B.S., Rago C., Velculescu V., Traverso G., Romans K.E., Montegomery E., Lal A., Riggins G.J., Lengauer C., Vogelstein B., et al. Genes expressed in human tumor endothelium. Science. 2000; 289: 1197-1202. doi: 10.1126/science.289.5482.1197.). It was subsequently found that TEM8 also known as anthrax toxin receptor 1(ANTXR1), is also a receptor for anthrax toxin, sharing 58% homology with another anthrax toxin receptor, CMG2 (ANTXR2).

There are five splice variants of TEM8, V1, V2, V3, V4, and V5, which are identical in N-terminal sequence and differ primarily in the length of C-terminal splicing (Vargas M., Karamsetty R., Leppla S.H., Chaudry G.J. Broad Expression Analysis of Human ANTXR1/TEM8 Transcripts Reveals Differential Expression and Novel Splizce Variants. PLoS ONE. 2012; 7: e43174. doi: 10.1371/annotation/cebf633f-19e7-496b-b370-c0f1b1aea888.). The major splice variant V1 is the full-length 564-aa TEM8 in which amino acids 322-342 belong to a transmembrane domain. V2, V3, V4, and V5 have 368, 333, 528, and 358 amino acids, respectively. Therefore, V2 and V4 possess transmembrane domains and, like V1, are membrane proteins, whereas V3 and V5 lack the TM domains and are soluble proteins. Furthermore, TEM8 is highly evolutionarily conserved, with identical amino acid sequences between humans and cynomolgus monkeys, as well as completely identical between mice and rats. Human and murine TEM8 share 98.9% homology.

There is a significant high expression of TEM8 in a variety of tumor tissues, including breast cancer, colorectal cancer, lung cancer, esophageal cancer, gastric cancer, pancreatic cancer, ovarian cancer, and the like. Further studies revealed that TEM8 is predominantly expressed on tumor-associated endothelial cells, pericytes, and tumor-associated fibroblasts (Szot C, et al. Tumor stroma-targeted antibody-drug conjugate triggers localized anticancer drug release. J Clin Invest. 2018; 128: 2927-2943. doi: 10.1172/JCI120481.). TEM8 has also been reported to be expressed by a subset of tumor cells, and its abundance in tumor-associated fibroblasts is further elevated under nutritional deprivation in the tumor microenvironment . Furthermore, these solid tumors frequently develop a pronounced stromal reaction, termed "desmoplastic stroma" or "reactive stroma", which accounts for 20-60% of the total tumor mass and is characterized by the presence of abundant stromal cells and dense extracellular matrix (ECM).

By virtue of the binding of TEM8 to collagen I and VI, tumor-associated fibroblasts migrate along the tumor stromal collagen fibers (Nanda A., Carson-Walter E.B., Seaman S., Barber T.D., Stampfl J., Singh S., Vogelstein B., Kinzler K.W., St Croix B. TEM8 interacts with the cleaved C5 domain of collagen alpha 3(VI) Cancer Res. 2004;64:817-820. doi: 10.1158/0008-5472.can-03-2408). It has also been recently reported that, in the nutrient-deficient tumor microenvironment, TEM8 mediates the uptake of collagen fibrils in tumor-associated fibroblasts, and these cells processed the collagen fibrils into glutamine, thereby promoting tumor cell growth (Hsu K.-S., Dunleavey J.M., Szot C., Yang L., Hilton M.B., Morris K., Seaman S., Feng Y., Lutz E.M., Koogle R., et al. Cancer cell survival depends on collagen uptake into tumor-associated stroma. Nat. Commun. 2022;13:7078. doi: 10.1038/s41467-022-34643-5). Tumor cells grow significantly slowly in TEM8-knockout mice (Nanda A., Carson-Walter E.B., Seaman S., Barber T.D., Stampfl J., Singh S., Vogelstein B., Kinzler K.W., St Croix B. TEM8 interacts with the cleaved C5 domain of collagen alpha 3(VI) Cancer Res. 2004;64:817-820. doi: 10.1158/0008-5472.can-03-2408). In addition, the blockade of the binding of TEM8 and collagen fibers with TEM8-specific antibody shows anti-tumor effect in various tumor models such as melanoma, lung cancer, colorectal cancer, and the like.

In contrast to VEGF, VEGFRs, and other angiogenic factors, TEM8 has no influence on physiological angiogenesis. TEM8-knockout mice display no overt defects in vessel development and wound healing. (Cullen M, Seaman S, Chaudhary A, Yang MY, Hilton MB, Logsdon D, et al. Host-Derived Tumor Endothelial Marker 8 (TEM8) Promotes the Growth of Melanoma. Cancer Res (2009) 69:6021-6. doi: 10.1158/0008-5472.CAN-09-1086). The above studies indicate that TEM8 has the potential to become an emerging target for tumor therapy, and antibodies with high affinity and high specificity for TEM8 held great promise for clinical applications. According to the present invention, a TEM8-specific antibody is discovered and identified via a fully human phage library, and the application prospect of the antibody is explored.

Although some anti-TEM8 antibodies have been developed, there remains a need to further development of TEM8 antibodies based on the prior art, in particular for the development of anti-human TEM8 antibodies with high specificity, high biological activity, high endocytosis capacity, and/or that can be used to prepare ADCs and CAR-Ts.

### SUMMARY OF THE INVENTION

The present invention discloses a TEM8-targeted antibody or antigen-binding fragment thereof, and use thereof.

The present invention thus provides a novel TEM8-binding antibody and antigen-binding fragment thereof.

In some embodiments, the anti-TEM8 antibody of the present invention has one or more or all of the following properties:
(1) being capable of binding with high affinity to TEM8, e.g., to TEM8 of human, cynomolgus monkey, rat, or mouse;
(2) being capable of binding with high affinity to TEM8 expressed on the cell membrane surface, e.g., to TEM8 of human, cynomolgus monkey, rat, or mouse;
(3) being capable of inducing endocytosis of the antibody or fragment thereof or a molecule comprising the antibody or fragment thereof (e.g., an ADC) by a TEM8-positive cell (e.g., a TEM8-overexpressing CHO cell);
(4) being capable of targeting and killing a TEM8-positive cell, such as a TEM8-positive tumor cell;
(5) being capable of treating a tumor, such as a cancer; or
(6) being suitable for constructing an antibody-drug conjugate (ADC) for targeting and killing TEM8-positive cells, such as TEM8-positive tumor cells, or for the treatment of tumors such as cancer.

In one embodiment, the present invention provides a nucleic acid encoding the antibody or a fragment thereof described herein, a vector comprising the nucleic acid, and a host cell comprising the vector.

In some embodiments, the present invention provides a method for the preparation of the antibody or a fragment thereof as described herein.

In some embodiments, the present invention provides a molecule comprising the antibodies as described herein, such as an antibody-drug conjugate, a pharmaceutical composition, and a combination product.

The present invention also provides a method of preventing or treating a TEM8-associated disease, e.g., a tumor such as a cancer, preferably a TEM8-positive tumor, e.g., a TEM8-positive cancer, in a subject using the antibody or antibody-drug conjugate thereof as described herein.

The present invention also relates to a method of detecting TEM8 in a sample.

### BRIEF DESCRIPTION OF THE DRAWINGS

FIG. 1 shows the construction of CHO stable cell lines of human and murine TEM8.
FIG. 2 shows the EC50 for 5 candidate molecules binding to human and mouse TEM8-overexpressing cells, as detected by FACS.
FIG. 3 shows the IC50 for endocytosis of 5 antibodies by human and mouse TEM8-overexpressing cells, respectively, as detected by a DT3C method.
FIG. 4 shows the in vitro killing IC50 of 5 MMAE-conjugated candidate molecules.
FIG. 5 shows the in vivo activity of 5 MMAE-conjugated candidate molecules in the HCT116 CDX model.
FIG. 6 shows the in vivo activity of naked antibody 6A6Q in the HCT116 CDX model.

### DETAILED DESCRIPTION OF THE INVENTION

For purposes of interpreting this specification, the following definitions will be used, and where appropriate, terms used in the singular may also include the plural, and vice versa. It is to be understood that this invention is not limited to the particular methodology, protocols, and reagents described herein, as these may vary. It is also to be understood that the terminology used herein is for the purpose of describing particular embodiments only, and is not intended to limit the scope of the present invention, which will be limited only by the appended claims. Unless otherwise specified, all technical and scientific terms used herein have the same meanings as those generally understood by a person of ordinary skill in the art to which the present invention belongs.

The term "about" when used in conjunction with a numerical value is intended to encompass the numerical value within a range having a lower limit that is 5% less than the numerical value specified, and an upper limit that is 5% greater than the numerical value specified.

As used herein, the term "and/or" means any one of the alternatives or two or more of the alternatives.

As used herein, the terms "comprising" or "including" mean including the recited elements, integers, or steps, but not excluding any other elements, integers, or steps. When the term "comprising" or "including" is used herein, unless otherwise indicated, it also encompasses the situation combining the stated elements, integers, or steps. For example, reference to an antibody variable region "comprising" a particular sequence is also intended to encompass antibody variable regions consisting of that particular sequence.

As used herein, the terms "anti," "binding," or "specific binding" mean that the binding to the target or antigen is selective and may be distinguished from unwanted or non-specific interactions. The ability of a binding site to bind to a particular target or antigen may be determined by flow cytometry or enzyme-linked immunosorbent assay (ELISA), or conventional binding assays known in the art, such as by radioimmunoassay (RIA) or bio-layer interferometry or MSD assay, or surface plasmon resonance (SPR).

The term "anti-TEM8 antibody", "anti-TEM8", "TEM8 antibody" or "antibody binding to TEM8" as used herein refers to an antibody that is capable of binding TEM8 (of primate, e.g., human or cynomolgus monkey, or mouse or rat) or a fragment thereof with sufficient affinity.

An "isolated" antibody or molecule is an antibody or molecule that has been separated from components of its natural environment. In some embodiments, the antibody or molecule is purified to greater than 95% or 99% purity as determined, for example, by electrophoresis (e.g., SDS-PAGE, IEF, capillary electrophoresis) or chromatography (e.g., ion exchange or reverse phase HPLC).

The terms "whole antibody" or "full-length antibody" are used interchangeably herein to refer to an antibody molecule having the structure of a native immunoglobulin molecule. In the case of a conventional four-chain IgG antibody, the full-length antibody comprises two heavy chains (H) and two light chains (L) interconnected by disulfide bonds. In the case of a heavy chain antibody having only heavy chains and lacking light chains, the full-length antibody comprises two heavy chains (H) interconnected by disulfide bonds. For conventional four-chain IgG antibodies, the full-length antibody heavy chain typically consists of a heavy chain variable region (abbreviated herein as VH) and a heavy chain constant region, wherein the heavy chain constant region comprises at least 3 domains: CH1, CH2, and CH3. A full-length antibody light chain consists of a light chain variable region (abbreviated herein as VL) and a light chain constant region, wherein the light chain constant region consists of one domain CL. Each heavy chain variable region VH and each light chain variable region consists of three CDRs and four FRs, arranged from amino-terminus to carboxy-terminus in the following order: FR1, CDR1, FR2, CDR2, FR3, CDR3, FR4. In some embodiments, the antibody according to the present invention is a full-length antibody, for example, a conventional four-chain IgG full-length antibody.

The term "antibody fragment" encompasses a portion of an intact antibody. In a preferred embodiment, the antibody fragment is an antigen-binding fragment. The term "antigen-binding fragment" of an antibody is a molecule distinct from a full-length antibody, which comprises a portion of the full-length antibody, but which binds to an antigen of the full-length antibody or competes for binding to an antigen with the full-length antibody (i.e., with the full-length antibody from which the antigen-binding fragment is derived). The antigen-binding fragments may be prepared by recombinant DNA techniques or by enzymatic or chemical cleavage of intact antibodies. Antigen-binding fragments include, but are not limited to, Fv, Fab, Fab', Fab'-SH, F(ab')2, dAb (domain antibodies), linear antibodies, single-chain antibodies (e.g., scFv), single-domain antibodies such as VHH, bivalent antibodies or fragments thereof, or camelid antibodies, diabody, single-domain antibodies (sdAb), and nanobodies. For example, Fab fragments may be obtained by papain digestion of full-length antibodies. In addition, digestion of the complete antibody by pepsin below the disulfide bond in the hinge region produces F(ab')2, which is a dimer of Fab' and is a bivalent antibody fragment. The F(ab')2 may be reduced under neutral conditions by disrupting the disulfide bond in the hinge region, thereby converting the F(ab')2 dimer into a Fab' monomer. The Fv fragment consists of the VL and VH domains of a single arm of an antibody. The two domains of the Fv fragment, VL and VH, may be encoded by separate genes, but it is also possible to link the two domains by recombinant methods using a synthetic linker peptide so that they are produced as a single protein chain in which the VL and VH regions pair to form a single-chain Fv (scFv).

A "complementary determining region" or "CDR region" or "CDR" is a region of an antibody variable domain that is highly variable in sequence and forms a structurally defined loop ("hypervariable loop") and/or includes an antigen-contacting residue ("antigen-contacting point"). The CDRs are primarily responsible for the binding to antigenic epitopes. The CDRs of the heavy chain and light chain are commonly referred to as CDR1, CDR2, and CDR3, numbered sequentially from the N-terminus. CDRs located within the variable domain of the heavy chain of an antibody are referred to as HCDR1, HCDR2, and HCDR3, while CDRs located within the variable domain of the light chain of an antibody are referred to as LCDR1, LCDR2, and LCDR3. In a given amino acid sequence of a light chain variable region or heavy chain variable region, the exact amino acid sequence boundaries of each CDR may be determined using any one or a combination of a number of well-known antibody CDR designating schemes, including, for example, Chothia scheme based on the three-dimensional structure of antibodies and the topology of CDR loops (Chothia et al., (1989) Nature 342: 877-883, Al-Lazikani et al., "Standard conformations for the canonical structures of immunoglobulins", Journal of Molecular Biology, 273, 927-948 (1997)); Kabat scheme based on antibody sequence variability (Accessing the Kabat antibody sequence database by computer; Martin AC; Proteins, 1996 May;25(1):130-3; doi: 10.1002/(SICI)1097-0134(199605)25:1<130::AID-PROT11>3.0.CO;2-L.PMID: 8727325); AbM scheme (University of Bath); Contact scheme (University College London); International ImMunoGeneTics database (IMGT) scheme (IMGT, the international ImMunoGeneTics information system, http://imgt.cines.fr; Lefranc MP; Novartis Found Symp. 2003; 254:126-36; discussion 136-42, 216-22, 250-2. PMID: 14712935 Review); and North CDR definition scheme based on affinity propagation clustering using a large number of crystal structures.

In some embodiments, the CDRs of the heavy chain variable region of the antibodies of the present invention are determined according to the Kabat or IMGT scheme. In some embodiments, the CDRs of the light chain variable region of the antibodies of the present invention are determined according to the Kabat or IMGT scheme.

Unless otherwise indicated herein, the amino acid residues in the Fc or heavy chain constant region are numbered according to the EU numbering system (also known as the EU index) as described in Kabat et al., Sequences of Proteins of Immunological Interes, 5th ed., Public Health Service, National Institutes of Health, Bethesda, MD, 1991, NIH Publication 91-3242; the amino acid residues of the light chain variable region and the heavy chain variable region are numbered according to the Kabat numbering system (also known as the Kabat index) as described in Kabat et al., Sequences of Proteins of Immunological Interes, 5th ed., Public Health Service, National Institutes of Health, Bethesda, MD, 1991, NIH Publication 91-3242.

The terms "amino acid substitution" or "amino acid replacement" are used interchangeably herein to refer to the replacement of at least one amino acid residue in a predetermined parent amino acid sequence with different "substituted" amino acid residues. The substituted residue or residues may be "naturally occurring amino acid residues" (i.e., encoded by the genetic code) and are selected from the group consisting of: alanine (Ala); arginine (Arg); asparagine (Asn); aspartic acid (Asp); cysteine (Cys); glutamine (Gln); glutamic acid (Glu); glycine (Gly); histidine (His); isoleucine (Ile): leucine (Leu); lysine (Lys); methionine (Met); phenylalanine (Phe); proline (Pro); serine (Ser); threonine (Thr); tryptophan (Trp); tyrosine (Tyr); and valine (Val). Substitutions with one or more non-naturally occurring amino acid residues are also encompassed within the definition of amino acid substitution herein. "Non-naturally occurring amino acid residue" refers to a residue that is capable of covalently binding adjacent amino acid residues in a polypeptide chain, in addition to those naturally occurring amino acid residues listed above. Examples of non-naturally occurring amino acid residues include norleucine, ornithine, norvaline, homoserine, Aib, and other amino acid residue analogs.

"Conservative alterations" as described herein include substitutions, deletions, or additions to the polypeptide sequence that do not substantially alter the desired functional activity of the polypeptide sequence. In some embodiments, the conservative alterations are conservative substitutions. "Conservative substitution" refers to a substitution of one amino acid with another within the same class, e.g., a substitution of one acidic amino acid with another acidic amino acid, a substitution of one basic amino acid with another basic amino acid, or a substitution of one neutral amino acid with another neutral amino acid. For example, conservative substitutions often result in the substitution of an amino acid with a chemically similar amino acid. Conservative substitution tables that provide functionally similar amino acids are well known in the art. The following list 8 groups of amino acids containing conservative substitutions for each other: 1) alanine (a), glycine (G); 2) aspartic acid (D), glutamic acid (E); 3) asparagine (N), glutamine (Q); 4) arginine (R), lysine (K); 5) isoleucine (I), leucine (L), methionine (M), valine (V); 6) phenylalanine (F), tyrosine (Y), tryptophan (W); 7) serine (S), threonine (T); and 8) cysteine (C), methionine (M). In some embodiments, the term "conservative alterations" as applied to an antibody molecule amino acid sequence is used to refer to an amino acid modification that does not significantly affect or alter the binding characteristics of the antibody molecule of the present invention comprising the amino acid sequence to the antigen of interest. For example, conservatively altered variants will retain at least 80%, 85%, 90%, 95%, 98%, 99% or more, e.g., 100-110% or more, binding affinity for the antigen of interest relative to the parent antibody.

The term "therapeutic agent" as used herein encompasses any substance effective in preventing or treating tumors, such as cancer, including chemotherapeutic agents, cytokines, cytotoxic agents, other antibodies, small molecule drugs, or immunomodulators (e.g., immunosuppressants).

A "chemotherapeutic agent" includes a chemical compound useful in the treatment of cancer or diseases of the immune system.

The term "small molecule drug" refers to an organic compound with low molecular weight capable of modulating biological processes. A "small molecule" is defined as a molecule having a molecular weight of less than 10kD, typically less than 2kD, and preferably less than 1 kD. Small molecules include, but are not limited to, inorganic molecules, organic molecules, organic molecules containing inorganic components, molecules containing radioactive atoms, synthetic molecules, peptidomimetics, and antibody mimetics. As a therapeutic agent, small molecules may be more permeable to cells, less susceptible to degradation, and less prone to eliciting an immune response than large molecules.

The term "immunomodulator" as used herein refers to a native or synthetic active agent or drug that inhibits or modulates an immune response. The immune response may be a humoral response or a cellular response. The immunomodulator includes an immunosuppressant. In some embodiments, the immunomodulator of the present invention comprises an immune checkpoint inhibitor or an immune checkpoint agonist.

An "isolated nucleic acid encoding an anti-TEM8 antibody or fragment thereof" refers to one or more nucleic acid molecules encoding the heavy or light chain (or fragment thereof, e.g., a heavy chain variable region or a light chain variable region) of an antibody, including such nucleic acid molecules in a single vector or separate vectors, as well as such nucleic acid molecules present at one or more locations in a host cell.

"Percent identity (%)" of an amino acid sequence refers to the percentage of amino acid residues in a candidate sequence that are identical to the amino acid residues of the specific amino acid sequence shown in this specification, after aligning the candidate sequence with the specific amino acid sequence shown in this specification and introducing gaps, if necessary, to achieve the maximum percent sequence identity, and without taking into account any conservative substitutions as part of sequence identity. In some embodiments, the present invention contemplates variants of an antibody molecule of the present invention that have a substantial degree of identity, e.g., at least 80%, 85%, 90%, 95%, 97%, 98%, or 99% or more identity, relative to the antibody molecule and sequence thereof specifically disclosed herein. The variant may comprise conservative alterations.

The term "pharmaceutically acceptable auxiliary material" refers to a diluent, adjuvant (e.g., Freund's adjuvant (complete and incomplete)), excipient, carrier, or stabilizer, etc., with which the active substance is administered.

As used herein, the term "label" refers to a compound or composition that is directly or indirectly conjugated or fused to an agent, such as a polynucleotide probe or antibody, and facilitates the detection of the agent to which it is conjugated or fused. The label may be detectable by itself (e.g., a radioisotope label or a fluorescent label) or, in the case of an enzymatic label, may catalyze chemical alteration of the detectable substrate compound or composition. The term is intended to encompass direct labeling of the probe or antibody by coupling (i.e., physically linking) a detectable substance to the probe or antibody, as well as indirect labeling of the probe or antibody by reaction with another agent that is directly labeled.

The term "combination therapy" refers to the administration of two or more therapeutic agents or treatment modalities to treat the diseases described herein. Such administration includes coadministering the therapeutic agents in a substantially simultaneous manner, for example, in a single capsule having a fixed ratio of active ingredients. Alternatively, such administration includes coadministration of each active ingredient in multiple or separate containers (e.g., tablets, capsules, powders, and liquids). The powder and/or liquid may be reconstituted or diluted to the desired dosage prior to administration. In addition, such administration also includes the use of each type of therapeutic agent at approximately the same time or at different times in a sequential manner. In either case, the treatment regimen will provide a beneficial effect of the pharmaceutical combination in treating the disorders or conditions described herein.

As used herein, the term "treating" (or "treat" or "treatment") refers to slowing, interrupting, arresting, relieving, stopping, lowering, or reversing the onset of the symptoms, complications, or biochemical indicators of a disease, relieving the symptoms, or preventing or inhibiting the further development of the disease, condition, or disorder.

As used herein, "preventing" (or "prevent" or "prevention") includes the inhibition of the development or progression of symptoms of a disease or disorder, or a specific disease or disorder.

A "subject/patient/individual sample" refers to a collection of cells or fluids obtained from a patient or subject. The source of the tissue or cell sample may be a solid tissue, such as from fresh, frozen and/or preserved organs or tissue samples or biopsy samples or puncture samples; blood or any blood component; body fluids such as tears, vitreous fluid, cerebrospinal fluid, amnion fluid (amniotic fluid), peritoneal fluid (ascites fluid), or interstitial fluid; cells from a subject at any time of gestation or development. Tissue samples may contain compounds that are not naturally intermixed with tissue in nature, such as preservatives, anticoagulants, buffers, fixatives, nutrients, antibiotics, etc.

In a first aspect of the present invention, there is provided an antibody specifically binding to TEM8 or a fragment thereof (e.g., an antigen-binding fragment).

In some embodiments, the anti-TEM8 antibody or antigen-binding fragment thereof of the present invention comprises 3 complementarity-determining regions (HCDRs) from the heavy chain variable region, HCDR1, HCDR2, and HCDR3. In some embodiments, the anti-TEM8 antibody or antigen-binding fragment thereof of the present invention comprises 3 complementarity-determining regions (LCDRs) from the light chain variable region, LCDR1, LCDR2, and LCDR3. In some embodiments, the anti-TEM8 antibody or antigen-binding fragment thereof of the present invention comprises 3 complementarity-determining regions (HCDRs) from the heavy chain variable region and 3 complementarity-determining regions (LCDRs) from the light chain variable region.

In some aspects, the anti-TEM8 antibody or antigen-binding fragment thereof of the present invention comprises a heavy chain variable region (VH). In some aspects, the anti-TEM8 antibody or antigen-binding fragment thereof of the present invention comprises a light chain variable region (VL). In some aspects, the anti-TEM8 antibody or antigen-binding fragment thereof of the present invention comprises a heavy chain variable region (VH) and a light chain variable region (VL). In some embodiments, the heavy chain variable region comprises 3 complementarity-determining regions (HCDRs) from the heavy chain variable region, HCDR1, HCDR2, and HCDR3. In some embodiments, the light chain variable region comprises 3 complementarity-determining regions (LCDRs) from the light chain variable region, LCDR1, LCDR2, and LCDR3.

In some embodiments, the heavy chain variable region of the present invention
(i) comprises or consists of an amino acid sequence having at least 90%, 91%, 92%, 93%, 94%, 95%, 96%, 97%, 98%, or 99% identity to the amino acid sequence selected from the group consisting of SEQ ID NOs: 1, 14, 27, 42, or 55; or
(ii) comprises or consists of an amino acid sequence selected from the group consisting of SEQ ID NOs: 1, 14, 27, 42, or 55; or
(iii) comprises or consists of an amino acid sequence having 1 or more (preferably not more than 10, more preferably not more than 5, 4, 3, 2, 1) amino acid alterations (e.g., conservative alterations, such as amino acid substitutions, more preferably conservative amino acid substitutions) as compared to the amino acid sequence selected from the group consisting of SEQ ID NOs: 1, 14, 27, 42, or 55, preferably the amino acid alterations do not occur in the CDR regions.

In some embodiments, the light chain variable region of the present invention
(i) comprises or consists of an amino acid sequence having at least 90%, 91%, 92%, 93%, 94%, 95%, 96%, 97%, 98%, or 99% identity to the amino acid sequence selected from the group consisting of SEQ ID NOs: 2, 15, 28, 40, 43, or 56; or
(ii) comprises or consists of an amino acid sequence selected from the group consisting of SEQ ID NOs: 2, 15, 28, 40, 43, or 56; or
(iii) comprises or consists of an amino acid sequence having 1 or more (preferably not more than 10, more preferably not more than 5, 4, 3, 2, 1) amino acid alterations (e.g., conservative alterations, such as amino acid substitutions, more preferably conservative amino acid substitutions) as compared to the amino acid sequence selected from the group consisting of SEQ ID NOs: 2, 15, 28, 40, 43, or 56, preferably the amino acid alterations do not occur in the CDR regions.

In some embodiments, the 3 complementarity-determining regions (HCDRs) from the heavy chain variable region of the present invention, HCDR1, HCDR2, and HCDR3, are selected from
(i) 3 complementarity-determining regions HCDR1, HCDR2, and HCDR3 contained in the VH as set forth in SEQ ID NOs: 1, 14, 27, 42, or 55; or
(ii) a sequence comprising at least one and no more than 5, 4, 3, 2, or 1 amino acid alterations (preferably amino acid substitution, preferably conservative substitution) in total in the three HCDR regions relative to any one of the sequence in (i),
wherein the HCDRs may be determined according to any CDR designating schemes, e.g., by an designating scheme of Kabat, AbM, Chothia, or IMGT, or a combination thereof, respectively, for example, the HCDRs are determined according to the IMGT or Kabat scheme, respectively.

In some embodiments, the 3 complementarity-determining regions (LCDRs) from the light chain variable region of the present invention, LCDR1, LCDR2, and LCDR3, are selected from
(i) 3 complementarity-determining regions LCDR1, LCDR2, and LCDR3 contained in the VL as set forth in SEQ ID NOs: 2, 15, 28, 40, 43, or 56;
   or
(ii) a sequence comprising at least one and no more than 5, 4, 3, 2, or 1 amino acid alterations (preferably amino acid substitution, preferably conservative substitution) in total in the three LCDR regions relative to any one of the sequence in (i),

wherein the LCDRs may be determined according to any CDR designating scheme, e.g. by an designating scheme of Kabat, AbM, Chothia or IMGT, or a combination thereof, respectively,
for example, the LCDRs are determined according to IMGT or Kabat scheme, respectively.

In some embodiment, HCDR1 determined based on IMGT scheme comprises or consists of an amino acid sequence as set forth in any one of SEQ ID NOs: 3, 16, 29, 44, or 57, or HCDR1 determined based on IMGT scheme comprises an amino acid sequence having one, two or three alterations (preferably amino acid substitutions, preferably conservative substitutions) as compared to the amino acid sequence as set forth in any one of SEQ ID NOs: 3, 16, 29, 44, or 57.

In some embodiment, HCDR1 determined based on Kabat scheme comprises or consists of an amino acid sequence as set forth in any one of SEQ ID NOs: 9, 22, 35, 50, or 63, or HCDR1 determined based on Kabat scheme comprises an amino acid sequence having one, two or three alterations (preferably amino acid substitutions, preferably conservative substitutions) as compared to the amino acid sequence as set forth in any one of SEQ ID NOs: 9, 22, 35, 50, or 63.

In some embodiment, HCDR2 determined based on IMGT scheme comprises or consists of an amino acid sequence as set forth in any one of SEQ ID NOs: 4, 17, 30, 45, or 58, or HCDR2 determined based on IMGT scheme comprises an amino acid sequence having one, two or three alterations (preferably amino acid substitutions, preferably conservative substitutions) as compared to the amino acid sequence as set forth in any one of SEQ ID NOs: 4, 17, 30, 45, or 58.

In some embodiment, HCDR2 determined based on Kabat scheme comprises or consists of an amino acid sequence as set forth in any one of SEQ ID NOs: 10, 23, 36, 51, or 64, or HCDR2 determined based on Kabat scheme comprises an amino acid sequence having one, two or three alterations (preferably amino acid substitutions, preferably conservative substitutions) as compared to the amino acid sequence as set forth in any one of SEQ ID NOs: 10, 23, 36, 51, or 64.

In some embodiment, HCDR3 determined based on IMGT scheme comprises or consists of an amino acid sequence as set forth in any one of SEQ ID NOs: 5, 18, 31, 46, or 59, or HCDR3 determined based on IMGT scheme comprises an amino acid sequence having one, two or three alterations (preferably amino acid substitutions, preferably conservative substitutions) as compared to the amino acid sequence as set forth in any one of SEQ ID NOs: 5, 18, 31, 46, or 59.

In some embodiment, HCDR3 determined based on Kabat scheme comprises or consists of an amino acid sequence as set forth in any one of SEQ ID NOs: 11, 24, 37, 52, or 65, or HCDR3 determined based on Kabat scheme comprises an amino acid sequence having one, two or three alterations (preferably amino acid substitutions, preferably conservative substitutions) as compared to the amino acid sequence as set forth in any one of SEQ ID NOs: 11, 24, 37, 52, or 65.

In some embodiment, LCDR1 determined based on IMGT scheme comprises or consists of an amino acid sequence as set forth in any one of SEQ ID NOs: 6, 19, 32, 47, or 60, or LCDR1 determined based on IMGT scheme comprises an amino acid sequence having one, two or three alterations (preferably amino acid substitutions, preferably conservative substitutions) as compared to the amino acid sequence as set forth in any one of SEQ ID NOs: 6, 19, 32, 47, or 60.

In some embodiment, LCDR1 determined based on Kabat scheme comprises or consists of an amino acid sequence as set forth in any one of SEQ ID NOs: 12, 25, 38, 53, or 66, or LCDR1 determined based on Kabat scheme comprises an amino acid sequence having one, two or three alterations (preferably amino acid substitutions, preferably conservative substitutions) as compared to the amino acid sequence as set forth in any one of SEQ ID NOs: 12, 25, 38, 53, or 66.

In some embodiment, LCDR2 determined based on IMGT scheme comprises or consists of an amino acid sequence as set forth in any one of SEQ ID NOs: 7, 20, 33, 48, or 61, or LCDR2 determined based on IMGT scheme comprises an amino acid sequence having one, two or three alterations (preferably amino acid substitutions, preferably conservative substitutions) as compared to the amino acid sequence as set forth in any one of SEQ ID NOs: 7, 20, 33, 48, or 61.

In some embodiment, LCDR2 determined based on Kabat scheme comprises or consists of an amino acid sequence as set forth in any one of SEQ ID NOs: 13, 26, 39, 54, or 67, or LCDR2 determined based on Kabat scheme comprises an amino acid sequence having one, two or three alterations (preferably amino acid substitutions, preferably conservative substitutions) as compared to the amino acid sequence as set forth in any one of SEQ ID NOs: 13, 26, 39, 54, or 67.

In some embodiment, LCDR3 determined based on IMGT scheme comprises or consists of an amino acid sequence as set forth in any one of SEQ ID NOs: 8, 21, 34, 41, 49, or 62, or LCDR3 determined based on IMGT scheme comprises an amino acid sequence having one, two or three alterations (preferably amino acid substitutions, preferably conservative substitutions) as compared to the amino acid sequence as set forth in any one of SEQ ID NOs: 8, 21, 34, 41, 49, or 62.

In some embodiment, LCDR3 determined based on Kabat scheme comprises or consists of an amino acid sequence as set forth in any one of SEQ ID NOs: 8, 21, 34, 41, 49, or 62, or LCDR3 determined based on Kabat scheme comprises an amino acid sequence having one, two or three alterations (preferably amino acid substitutions, preferably conservative substitutions) as compared to the amino acid sequence as set forth in any one of SEQ ID NOs: 8, 21, 34, 41, 49, or 62.

In some specific embodiments of the present invention, the anti-TEM8 antibody or antigen-binding fragment thereof of the present invention comprises:
1) 3 complementarity-determining regions HCDR1, HCDR2, and HCDR3 contained in VH as set forth in SEQ ID NO: 1, and 3 complementarity-determining regions LCDR1, LCDR2, and LCDR3 contained in VL as set forth in SEQ ID NO: 2;
2) 3 complementarity-determining regions HCDR1, HCDR2, and HCDR3 contained in VH as set forth in SEQ ID NO: 14, and 3 complementarity-determining regions LCDR1, LCDR2, and LCDR3 contained in VL as set forth in SEQ ID NO: 15;
3) 3 complementarity-determining regions HCDR1, HCDR2, and HCDR3 contained in VH as set forth in SEQ ID NO: 27, and 3 complementarity-determining regions LCDR1, LCDR2, and LCDR3 contained in VL as set forth in SEQ ID NO: 28 or 40;
4) 3 complementarity-determining regions HCDR1, HCDR2, and HCDR3 contained in VH as set forth in SEQ ID NO: 42, and 3 complementarity-determining regions LCDR1, LCDR2, and LCDR3 contained in VL as set forth in SEQ ID NO: 43; or
5) 3 complementarity-determining regions HCDR1, HCDR2, and HCDR3 contained in VH as set forth in SEQ ID NO: 55, and 3 complementarity-determining regions LCDR1, LCDR2, and LCDR3 contained in VL as set forth in SEQ ID NO: 56;
for example, wherein the HCDR and the LCDR are determined according to the IMGT or Kabat scheme.

In some specific embodiments of the present invention, the anti-TEM8 antibody or antigen-binding fragment thereof of the present invention comprises: 3 complementarity-determining regions HCDR1, HCDR2, and HCDR3 of the heavy chain variable region and 3 complementarity-determining regions LCDR1, LCDR2, and LCDR3 of the light chain variable region, wherein the HCDR1, HCDR2, HCDR3, LCDR1, LCDR2, and LCDR3 are based on the IMGT scheme, comprising or consisting of, respectively, the amino acid sequence as set forth in SEQ ID NOs in the table below:

| HCDR1 (IMGT) comprising or consisting of the SEQ ID NOs below: | HCDR2 (IMGT) comprising or consisting of the SEQ ID NOs below: | HCDR3 (IMGT) comprising or consisting of the SEQ ID NOs below: | LCDR1 (IMGT) comprising or consisting of the SEQ ID NOs below: | LCDR2 (IMGT) comprising or consisting of the SEQ ID NOs below: | LCDR3 (IMGT) comprising or consisting of the SEQ ID NOs below: |
|---|---|---|---|---|---|
| 3 | 4 | 5 | 6 | 7 | 8 |
| 16 | 17 | 18 | 19 | 20 | 21 |
| 29 | 30 | 31 | 32 | 33 | 34 |
| 29 | 30 | 31 | 32 | 33 | 41 |
| 44 | 45 | 46 | 47 | 48 | 49 |
| 57 | 58 | 59 | 60 | 61 | 62 |

or wherein the HCDR1, HCDR2, HCDR3, LCDR1, LCDR2, and LCDR3 are based on the Kabat scheme, comprising or consisting of, respectively, an amino acid sequence as set forth in SEQ ID NOs in the table below:

| HCDR1 (Kabat) comprising or consisting of the SEQ ID NOs below: | HCDR2 (Kabat) comprising or consisting of the SEQ ID NOs below: | HCDR3 (Kabat) comprising or consisting of the SEQ ID NOs below: | LCDR1 (Kabat) comprising or consisting of the SEQ ID NOs below: | LCDR2 (Kabat) comprising or consisting of the SEQ ID NOs below: | LCDR3 (Kabat) comprising or consisting of the SEQ ID NOs below: |
|---|---|---|---|---|---|
| 9 | 10 | 11 | 12 | 13 | 8 |
| 22 | 23 | 24 | 25 | 26 | 21 |
| 35 | 36 | 37 | 38 | 39 | 34 |
| 35 | 36 | 37 | 38 | 39 | 41 |
| 50 | 51 | 52 | 53 | 54 | 49 |
| 63 | 64 | 65 | 66 | 67 | 62 |

In some specific embodiments of the present invention, the anti-TEM8 antibody or antigen-binding fragment thereof of the present invention comprises a VH and a VL, wherein the VH and VL comprise or consist of, respectively, an amino acid sequence as set forth in SEQ ID NOs in the table below, or an amino acid sequence having at least 90%, 91%, 92%, 93%, 94%, 95%, 96%, 97%, 98%, or 99% identity to the amino acid sequence as set forth in SEQ ID NOs as shown in the table below:

| VH | VL |
|---|---|
| SEQ ID NO: 1 | SEQ ID NO: 2 |
| SEQ ID NO: 14 | SEQ ID NO: 15 |
| SEQ ID NO: 27 | SEQ ID NO: 28 or SEQ ID NO: 40 |
| SEQ ID NO: 42 | SEQ ID NO: 43 |
| SEQ ID NO: 55 | SEQ ID NO: 56 |

In a preferred embodiment, the present invention provides an anti-TEM8 antibody or antigen-binding fragment thereof, comprising a heavy chain variable region and a light chain variable region, wherein the heavy chain variable region and light chain variable region comprise or consist of, respectively, the amino acid sequence as set forth in SEQ ID NOs as shown in the table below:

| VH | VL |
|---|---|
| SEQ ID NO: 1 | SEQ ID NO: 2 |
| SEQ ID NO: 14 | SEQ ID NO: 15 |
| SEQ ID NO: 27 | SEQ ID NO: 28 or SEQ ID NO: 40 |
| SEQ ID NO: 42 | SEQ ID NO: 43 |
| SEQ ID NO: 55 | SEQ ID NO: 56 |

In some embodiments, the anti-TEM8 antibody or antigen-binding fragment thereof of the present invention further comprises an antibody heavy chain constant region. In some embodiments, the anti-TEM8 antibody or antigen-binding fragment thereof of the present invention further comprises an antibody light chain constant region. In some embodiments, the anti-TEM8 antibody or antigen-binding fragment thereof of the present invention further comprises a heavy chain constant region and a light chain constant region.

In some embodiments, the antibody heavy chain constant region of the present invention is the heavy chain constant region of IgG1, IgG2, IgG3, or IgG4, preferably the heavy chain constant region of IgG1. In some embodiments, the antibody light chain constant region of the present invention is a Lambda or Kappa light chain constant region.

In some preferred embodiments, the antibody heavy chain constant region of the present invention
(i) comprises or consists of an amino acid sequence having at least 85%, 90%, 91%, 92%, 93%, 94%, 95%, 96%, 97%, 98%, or 99% identity to the amino acid sequence selected from SEQ ID NO:71;
(ii) comprises or consists of an amino acid sequence selected from SEQ ID NO: 71; or
(iii) comprises or consists of an amino acid sequence having 1 or more (preferably not more than or 20 or 10, more preferably not more than 5, 4, 3, 2, 1) amino acid alterations (e.g., conservative alterations, such as amino acid substitutions, more preferably conservative amino acid substitutions) as compared to the amino acid sequence selected from SEQ ID NO: 71.

In some embodiments, the antibody light chain constant region of the present invention
(i) comprises or consists of an amino acid sequence having at least 85%, 90%, 91%, 92%, 93%, 94%, 95%, 96%, 97%, 98%, or 99% identity to the amino acid sequence selected from the group consisting of SEQ ID NO: 72 or 73;
(ii) comprises or consists of an amino acid sequence selected from the group consisting of SEQ ID NO: 72 or 73; or
(iii) comprises or consists of an amino acid sequence having 1 or more (preferably not more than or 20 or 10, more preferably not more than 5, 4, 3, 2, 1) amino acid alterations (e.g., conservative alterations, such as amino acid substitutions, more preferably conservative amino acid substitutions) as compared to the amino acid sequence selected from the group consisting of SEQ ID NO: 72 or 73.

In some embodiments, the antibody or fragment thereof of the present invention comprises a heavy chain constant region comprising or consisting of the amino acid sequence as set forth in SEQ ID NO:71, and a light chain constant region comprising or consisting of the amino acid sequence as set forth in SEQ ID NO:72 or 73.

In some embodiments, the anti-TEM8 antibody or antigen-binding fragment thereof of the present invention comprises an antibody heavy chain (HC). In some embodiments, the anti-TEM8 antibody or antigen-binding fragment thereof of the present invention further comprises an antibody light chain (LC). In some embodiments, the anti-TEM8 antibody or antigen-binding fragment thereof of the present invention comprises a heavy chain and a light chain. In some embodiments, the antibody heavy chain of the present invention comprises or consists of an antibody heavy chain variable region and an antibody heavy chain constant region. In some embodiments, the antibody light chain of the present invention comprises or consists of an antibody light chain variable region and an antibody light chain constant region. In some embodiments, the antibody of the present invention comprises or consists of two heavy chains and two light chains, e.g., the two heavy chains are identical and the two light chains are identical.

In some embodiments, the antibody of the present invention comprises a heavy chain comprising a heavy chain variable region and a heavy chain constant region, and a light chain comprising a light chain variable region and a light chain constant region, wherein
(i). the heavy chain variable region comprises or consists of the amino acid sequence as set forth in SEQ ID NO: 1, or an amino acid sequence having at least 90%, 91%, 92%, 93%, 94%, 95%, 96%, 97%, 98%, or 99% identity thereto; the light chain variable region comprises or consists of the amino acid sequence as set forth in SEQ ID NO:2, or an amino acid sequence having at least 90%, 91%, 92%, 93%, 94%, 95%, 96%, 97%, 98%, or 99% identity thereto; the heavy chain constant region comprises or consists of the amino acid sequence as set forth in SEQ ID NO:71, or an amino acid sequence having at least 90%, 91%, 92%, 93%, 94%, 95%, 96%, 97%, 98%, or 99% identity thereto; and the light chain constant region comprises or consists of the amino acid sequence as set forth in SEQ ID NO:72, or an amino acid sequence having at least 90%, 91%, 92%, 93%, 94%, 95%, 96%, 97%, 98%, or 99% identity thereto;
(ii). the heavy chain variable region comprises or consists of the amino acid sequence as set forth in SEQ ID NO: 14, or an amino acid sequence having at least 90%, 91%, 92%, 93%, 94%, 95%, 96%, 97%, 98%, or 99% identity thereto; the light chain variable region comprises or consists of the amino acid sequence as set forth in SEQ ID NO: 15, or an amino acid sequence having at least 90%, 91%, 92%, 93%, 94%, 95%, 96%, 97%, 98%, or 99% identity thereto; the heavy chain constant region comprises or consists of the amino acid sequence as set forth in SEQ ID NO:71, or an amino acid sequence having at least 90%, 91%, 92%, 93%, 94%, 95%, 96%, 97%, 98%, or 99% identity thereto; and the light chain constant region comprises or consists of the amino acid sequence as set forth in SEQ ID NO:72, or an amino acid sequence having at least 90%, 91%, 92%, 93%, 94%, 95%, 96%, 97%, 98%, or 99% identity thereto;
(iii). the heavy chain variable region comprises or consists of the amino acid sequence as set forth in SEQ ID NO:27, or an amino acid sequence having at least 90%, 91%, 92%, 93%, 94%, 95%, 96%, 97%, 98%, or 99% identity thereto; the light chain variable region comprises or consists of the amino acid sequence as set forth in SEQ ID NO: 28 or 40, or an amino acid sequence having at least 90%, 91%, 92%, 93%, 94%, 95%, 96%, 97%, 98%, or 99% identity thereto; the heavy chain constant region comprises or consists of the amino acid sequence as set forth in SEQ ID NO:71, or an amino acid sequence having at least 90%, 91%, 92%, 93%, 94%, 95%, 96%, 97%, 98%, or 99% identity thereto; and the light chain constant region comprises or consists of the amino acid sequence as set forth in SEQ ID NO:73, or an amino acid sequence having at least 90%, 91%, 92%, 93%, 94%, 95%, 96%, 97%, 98%, or 99% identity thereto;
(iv). the heavy chain variable region comprises or consists of the amino acid sequence as set forth in SEQ ID NO:42, or an amino acid sequence having at least 90%, 91%, 92%, 93%, 94%, 95%, 96%, 97%, 98%, or 99% identity thereto;
   the light chain variable region comprises or consists of the amino acid sequence as set forth in SEQ ID NO: 43, or an amino acid sequence having at least 90%, 91%, 92%, 93%, 94%, 95%, 96%, 97%, 98%, or 99% identity thereto;
   the heavy chain constant region comprises or consists of the amino acid sequence as set forth in SEQ ID NO:71, or an amino acid sequence having at least 90%, 91%, 92%, 93%, 94%, 95%, 96%, 97%, 98%, or 99% identity thereto;
   and the light chain constant region comprises or consists of the amino acid sequence as set forth in SEQ ID NO:73, or an amino acid sequence having at least 90%, 91%, 92%, 93%, 94%, 95%, 96%, 97%, 98%, or 99% identity thereto; or
(v). the heavy chain variable region comprises or consists of the amino acid sequence as set forth in SEQ ID NO:55, or an amino acid sequence having at least 90%, 91%, 92%, 93%, 94%, 95%, 96%, 97%, 98%, or 99% identity thereto;
   the light chain variable region comprises or consists of the amino acid sequence as set forth in SEQ ID NO: 56, or an amino acid sequence having at least 90%, 91%, 92%, 93%, 94%, 95%, 96%, 97%, 98%, or 99% identity thereto;
   the heavy chain constant region comprises or consists of the amino acid sequence as set forth in SEQ ID NO:71, or an amino acid sequence having at least 90%, 91%, 92%, 93%, 94%, 95%, 96%, 97%, 98%, or 99% identity thereto; and the light chain constant region comprises or consists of the amino acid sequence as set forth in SEQ ID NO:73, or an amino acid sequence having at least 90%, 91%, 92%, 93%, 94%, 95%, 96%, 97%, 98%, or 99% identity thereto.

In some embodiments, the antibody of the present invention comprises a heavy chain comprising a heavy chain variable region and a heavy chain constant region, and a light chain comprising a light chain variable region and a light chain constant region, wherein
(vi). the heavy chain variable region comprises or consists of the amino acid sequence as set forth in SEQ ID NO:1, the light chain variable region comprises or consists of the amino acid sequence as set forth in SEQ ID NO:2, the heavy chain constant region comprises or consists of the amino acid sequence as set forth in SEQ ID NO:71, and the light chain constant region comprises or consists of the amino acid sequence as set forth in SEQ ID NO: 72;
(vii). the heavy chain variable region comprises or consists of the amino acid sequence as set forth in SEQ ID NO: 14, the light chain variable region comprises or consists of the amino acid sequence as set forth in SEQ ID NO: 15, the heavy chain constant region comprises or consists of the amino acid sequence as set forth in SEQ ID NO:71, and the light chain constant region comprises or consists of the amino acid sequence as set forth in SEQ ID NO: 72;
(viii). the heavy chain variable region comprises or consists of the amino acid sequence as set forth in SEQ ID NO:27, the light chain variable region comprises or consists of the amino acid sequence as set forth in SEQ ID NO: 28 or 40, the heavy chain constant region comprises or consists of the amino acid sequence as set forth in SEQ ID NO:71, and the light chain constant region comprises or consists of the amino acid sequence as set forth in SEQ ID NO: 73;
(ix). the heavy chain variable region comprises or consists of the amino acid sequence as set forth in SEQ ID NO:42, the light chain variable region comprises or consists of the amino acid sequence as set forth in SEQ ID NO: 43, the heavy chain constant region comprises or consists of the amino acid sequence as set forth in SEQ ID NO:71, and the light chain constant region comprises or consists of the amino acid sequence as set forth in SEQ ID NO: 73; or
(x). the heavy chain variable region comprises or consists of the amino acid sequence as set forth in SEQ ID NO:55, the light chain variable region comprises or consists of the amino acid sequence as set forth in SEQ ID NO:56, the heavy chain constant region comprises or consists of the amino acid sequence as set forth in SEQ ID NO:71, and the light chain constant region comprises or consists of the amino acid sequence as set forth in SEQ ID NO: 73.

In one embodiment of the present invention, the amino acid alterations described herein are conservative alterations. In one embodiment of the present invention, the amino acid alterations described herein include substitutions, insertions, or deletions of amino acids. Preferably, the amino acid alterations described herein are amino acid substitutions, preferably conservative substitutions.

In preferred embodiments, the amino acid alterations described herein occur in regions outside of CDR (e.g., in FR). More preferably, the amino acid changes described herein occur in regions outside the heavy chain variable region and/or outside the light chain variable region.

In some embodiments, the substitutions are conservative substitutions. "Conservative substitution" refers to a substitution of one amino acid with another within the same class, e.g., a substitution of one acidic amino acid with another acidic amino acid, a substitution of one basic amino acid with another basic amino acid, or a substitution of one neutral amino acid with another neutral amino acid.

In certain embodiments, the substitution occurs in the CDR region of the antibody. Typically, the resulting variant will have modifications (e.g., improvements) in certain biological properties (e.g., increased affinity) relative to the parent antibody and/or will have substantially retained certain biological properties of the parent antibody.

In some embodiments, the antibodies provided herein are modified to remove glycosylation sites. In some embodiments, the glycosylation site NRS of the antibody or fragment thereof of the present invention is removed, preferably by mutation, e.g., the removal of the glycosylation site is achieved by mutating NRS to QRS.

In certain embodiments, it may be desirable to produce cysteine-engineered antibodies, such as "thio MAb", in which one or more residues of the antibody are substituted with a cysteine residue.

In certain embodiments, the antibodies provided herein may be further modified to contain other non-protein moieties known and readily available in the art. Suitable moieties for antibody derivatization include, but are not limited to, water-soluble polymers. Non-limiting examples of water-soluble polymers include, but are not limited to, polyethylene glycol (PEG), ethylene glycol/propylene glycol copolymers, carboxymethyl cellulose, dextran, polyvinyl alcohol, polyvinyl pyrrolidone, poly-1,3-dioxane, poly-1,3,6-trioxane, ethylene/maleic anhydride copolymers, polyamino acids (homopolymers or random copolymers), and dextran or poly (n-vinyl pyrrolidone) polyethylene glycol, propylene glycol homopolymers, poly(propylene oxide/ethylene oxide) copolymers, polyoxyethylated polyols (e.g., glycerol), polyvinyl alcohol, and mixtures thereof.

In some embodiments, the anti-TEM8 antibody or antigen-binding fragment thereof of the present invention further includes an antibody or antigen-binding fragment having one or more of the following properties:
(1) exhibiting the same or similar binding affinity and/or specificity for TEM8 as any of the antibodies listed in the invention;
(2) inhibiting the binding of any of the antibodies listed in the invention to TEM8;
(3) binding to the same or an overlapping epitope as any of the antibodies shown in the invention; or
(4) competing for binding to TEM8 with any of the antibodies shown in the invention.

As defined herein, "an antibody that binds to the same or an overlapping epitope" as a reference antibody refers to an antibody that blocks 50%, 60%, 70%, 80%, 90%, or 95% or more of the binding of the reference antibody to its antigen in a competition assay, and conversely, a reference antibody blocks 50%, 60%, 70%, 80%, 90% or 95% or more of the binding of the antibody to its antigen in a competition assay.

As defined herein, an antibody that competes with a reference antibody for binding to its antigen refers to an antibody that blocks 50%, 60%, 70%, 80%, 90%, or 95% or more of the binding of the reference antibody to its antigen in a competition assay. Conversely, a reference antibody blocks 50%, 60%, 70%, 80%, 90%, or 95% or more of the binding of the antibody to its antigen in a competition assay. Numerous types of competitive binding assays may be used to determine whether one antibody competes with another, such as direct or indirect solid-phase radioimmunoassay (RIA), direct or indirect solid-phase enzyme immunoassay (EIA), sandwich competitive assay, bio-optical interferometry (e.g., Fortebio), or surface plasmon resonance (Biacore).

As defined herein, an antibody that inhibits (e.g., competitively inhibits) the binding of a reference antibody to its antigen refers to an antibody that inhibits 50%, 60%, 70%, 80%, 90%, or 95% or more of the binding of the reference antibody to its antigen. Conversely, a reference antibody inhibits 50%, 60%, 70%, 80%, 90% or 95% or more of the binding of the antibody to its antigen. The binding of an antibody to its antigen may be measured by affinity (e.g., equilibrium dissociation constant). Methods for determining affinity are known in the art.

An antibody that exhibits the same or similar binding affinity and/or specificity as a reference antibody refers to an antibody that is capable of having a binding affinity and/or specificity of at least 50%, 60%, 70%, 80%, 90% or 95% or more of that of the reference antibody. This may be determined by any method known in the art for determining binding affinity and/or specificity.

In some embodiments, the anti-TEM8 antibody is a monoclonal antibody.

In some embodiments, the anti-TEM8 antibody is a human antibody.

In some embodiments, the anti-TEM8 antibody is a full-length antibody.

In one embodiment, the anti-TEM8 antibody in the invention also encompasses an antibody fragment thereof, such as an antigen-binding fragment thereof. In some embodiments, the antibody fragment includes, but is not limited to, Fab, Fab', F(ab')2, Fv, single chain Fv, diabodies, single domain antibodies (sdAb), nanobodies, or sc(Fv)2.

In certain embodiments, the anti-TEM8 antibody molecule is in the form of a bispecific or multispecific antibody molecule. In one embodiment, a bispecific antibody may bind to two different epitopes of the TEM8 protein. In one embodiment, a bispecific antibody may bind to TEM8 and another protein. Thus, the anti-TEM8 antibody of the present invention also encompasses a multispecific antibody, e.g., a bispecific antibody, that specifically binds to TEM8 and other antigens.

Thus, in some embodiments, the present invention relates to a bispecific or multispecific antibody that specifically binds to TEM8 and one or more other antigens.

In a second aspect of the present invention, there is provided an antibody-drug conjugate comprising the anti-TEM8 antibody or antigen-binding fragment thereof of the present invention.

The antibody or fragment thereof of the present invention is capable of inducing endocytosis of the antibody or fragment thereof or a molecule comprising the antibody or fragment thereof by a TEM8-positive cell, and is therefore suitable for use in the construction of antibody-drug conjugates.

In some embodiments, the present invention provides an antibody-drug conjugate comprising any of the anti-TEM8 antibodies provided herein and additional substances, such as therapeutic agents or labels. In some embodiments, the therapeutic agent is a cytotoxic agent. In some embodiments, the therapeutic agent is MMAE.

In some embodiments, the antibody or antigen-binding fragment thereof of the present invention is linked to a cytotoxic agent (e.g., MMAE) via a linker to form an antibody-drug conjugate. In some embodiments, the linker is a Vc linker. In some embodiments, the antibody or antigen-binding fragment thereof of the present invention is linked to VcMMAE to form an antibody-drug conjugate.

In a third aspect of the present invention, there is provided a nucleic acid, e.g., an isolated nucleic acid molecule, encoding the anti-TEM8 antibody or fragment thereof of the present invention or any one of strands thereof.

For example, the nucleic acid of the present invention comprises a nucleic acid encoding the amino acid sequence selected from any one of SEQ ID NOs: 1, 2, 14, 15, 27, 28, 40, 42, 43, 55 or 56, or an amino acid sequence having at least 85%, 90%, 91%, 92%, 93%, 94%, 95%, 96%, 97%, 98%, or 99% identity to the amino acid sequence selected from any one of SEQ ID NOs: 1, 2, 14, 15, 27, 28, 40, 42, 43, 55 or 56. As will be appreciated by those skilled in the art, each antibody or polypeptide amino acid sequence may be encoded by a variety of nucleic acid sequences due to codon degeneracy. The nucleic acid sequences encoding the molecules of the present invention may be generated using methods well known in the art, for example, by de novo solid-phase DNA synthesis or by PCR amplification.

When expressed from a suitable expression vector, the polypeptide encoded by the nucleic acid is capable of exhibiting human (and/or mouse, rat, dog, or cynomolgus monkey) TEM8 antigen-binding capacity. For example, in some embodiments, the nucleic acid encoding the variable region of a heavy and/or light chain is operably linked in reading frame to a nucleic acid encoding the constant region of a heavy and/or light chain, resulting in the nucleic acid encoding the antibody heavy chain and/or light chain when expressed from a suitable expression vector.

For ease of production and purification, a secretory signal peptide, and/or a tag peptide to facilitate purification, such as a his tag, may be fused to the N-terminus of the heavy chain and/or light chain of the antibody.

In a fourth aspect of the present invention, there is provided one or more vectors comprising the nucleic acid of the present invention.

The term "vector" as used herein refers to a nucleic acid molecule capable of propagating another nucleic acid to which it is linked. The term includes vectors that are self-replicating nucleic acid structures, as well as vectors that are incorporated into the genome of a host cell into which they have been introduced. The term "expression vector" refers to a vector comprising a recombinant polynucleotide comprising expression control sequences operably linked to the nucleotide sequence to be expressed. The expression vector comprises sufficient cis-acting elements for expression; other elements for expression may be provided by the host cell or in an in vitro expression system. Expression vectors include all those known in the art, including cosmids, plasmids (e.g., naked or included in liposomes), and viruses (e.g., lentiviruses, retroviruses, adenoviruses, and adeno-associated viruses) into which recombinant polynucleotides are incorporated.

In one embodiment, the vector is an expression vector, such as a eukaryotic expression vector. The vector includes, but is not limited to, viruses, plasmids, cosmids, Lambda phages, or yeast artificial chromosomes (YACs). In one embodiment, the vector is a pCDNA vector, such as pCDNA3.4.

In a fifth aspect of the present invention, there is provided a host cell comprising a nucleic acid encoding an antibody or antigen-binding fragment thereof of the invention, or a vector comprising the nucleic acid.

The term "host cell" refers to a cell into which an exogenous polynucleotide has been introduced, including the progeny of such cells. Host cells include "transformants" and "transformed cells," which include the primary transformed cells and progeny derived therefrom, regardless of the number of passages. The progeny may not be completely identical to the parent cell in nucleic acid content, but may contain mutations. Mutant progeny having the same function or biological activity as screened or selected in the initially transformed cell are included herein. Host cells are any type of cell system that may be used to produce the antibody molecule of the present invention, including eukaryotic cells, such as mammalian cells, insect cells, yeast cells; and prokaryotic cells, such as *E. coli* cells. Host cells include cultured cells, as well as cells within transgenic animals, transgenic plants, or cultured plant tissue or animal tissue.

In one embodiment, the host cell is eukaryotic. In another embodiment, the host cell is selected from the group consisting of a yeast cell, a mammalian cell (e.g., a CHO cell, such as CHO-S or CHO-K or CHO-K1, or a 293 cell, such as a 293T, 293F, or HEK293 cell), or other cells suitable for making the antibody or fragment thereof. In one embodiment, the host cell is prokaryotic, for example, is bacterium, such as *E.coli.*

In one embodiment, the host cell is eukaryotic. In another embodiment, the host cell is selected from a yeast cell, a mammalian cell, or other cells suitable for making the antibody or a fragment thereof. For example, eukaryotic microorganisms such as mycelial fungi or yeasts are suitable cloning or expression hosts for vectors encoding antibodies. Vertebrate cells may also be used as hosts. For example, mammalian cell lines engineered to be suitable for growth in suspension may be used. Other examples of useful mammalian host cell lines are monkey kidney CV1 line transformed with SV40 (COS-7); human embryonic kidney lines (HEK293, 293F, or 293T cells), etc. Other useful mammalian host cell lines include Chinese hamster ovary (CHO) cells, including CHO-S, CHO-K, or CHO-K1 and the like; and myeloma cell lines such as Y0, NS0, and Sp2/0. Mammalian host cell lines suitable for the production of antibodies are known in the art.

In a sixth aspect of the present invention, the present invention provides a method of preparing an anti-TEM8 antibody or fragment thereof (preferably an antigen-binding fragment), wherein the method includes culturing the host cell under conditions suitable for expression of a nucleic acid encoding the antibody or fragment thereof (preferably an antigen-binding fragment) or any one or two strands thereof, and optionally isolating the antibody or fragment thereof (preferably an antigen-binding fragment). In a certain embodiment, the method further comprises recovering the anti-TEM8 antibody or fragment thereof (preferably an antigen-binding fragment) from the host cell.

The polynucleotide encoding the polypeptide chain of the antibody or antigen-binding fragment thereof of the present invention may be inserted into one or more vectors for further cloning and/or expression in a host cell. Methods well known to those skilled in the art may be used to construct expression vectors. Once an expression vector comprising one or more nucleic acid molecules of the present invention has been prepared for expression, the expression vector may be transfected or introduced into a suitable host cell. Various techniques may be used to achieve this purpose, for example, protoplast fusion, calcium phosphate precipitation, electroporation, retroviral transduction, viral transfection, biolistic, liposome-based transfection, or other conventional techniques.

Antibodies prepared as described herein may be purified by known existing techniques such as high-performance liquid chromatography, ion exchange chromatography, gel electrophoresis, affinity chromatography, such as Protein A, and size exclusion chromatography, etc. The actual conditions used to purify a particular protein will also depend on factors such as net charge, hydrophobicity, and hydrophilicity, and will be apparent to those skilled in the art.

The purity of the antibody molecules of the present invention may be determined by any of a variety of well-known analytical methods, including size exclusion chromatography, gel electrophoresis, and high-performance liquid chromatography, etc.

In a seventh aspect of the present invention, there are provided methods or assays for identifying, screening, or characterizing the physical/chemical properties and/or biological activity of the anti-TEM8 antibody provided herein.

In one aspect, the antigen-binding activity of the antibodies in the present invention is tested, e.g., by known methods such as Bio-layer interferometry, ELISA, etc. Binding to TEM8 may be determined using methods known in the art, for example, measured by using radioimmunoassay (RIA) or bio-layer interferometry (BLI) assay or electrochemiluminescence (ECL) assay or surface plasmon resonance (SPR), or flow cytometry (FCM, also known as fluorescence activated cell sorting (FACS)).

The present invention also provides assays for identifying anti-TEM8 antibodies that are biologically active. The biological activity is selected from the properties of the antibodies of the present invention and may include, for example, binding to TEM8 (e.g., binding to TEM8 of human, cynomolgus monkey, mouse, and/or rat), cellular endocytic activity, or killing activity on TEM8-positive cells, and the like.

For example, the binding activity of an antibody molecule of the present invention to TEM8 or a TEM8-expressing cell may be determined by methods known in the art, such as Fortebio, flow cytometry, Octet, or plasma resonance (Biacore), etc., or by the exemplary methods disclosed in Example 3 herein.

For example, the endocytic activity of an antibody molecule of the present invention against a cell (e.g., a TEM8-positive cell, e.g., a TEM8-overexpressing cell) may be determined by methods known in the art, e.g., DT3C, or the exemplary methods disclosed in Example 4 herein.

For example, the killing activity or anti-tumor activity of the antibody molecules of the present invention or antibody-drug conjugates comprising the same against a cell (e.g., a TEM8-positive cell, e.g., a tumor cell) may be determined by methods known in the art, e.g., cell killing assays, such as in vitro cell killing assays, or in vivo animal model experiments, or the exemplary methods disclosed in Examples 7 or 8, or 9 herein.

Cells used for any of the above in vitro assays include cell lines that naturally express TEM8 or that are engineered to express TEM8. Such cells also include cell lines that express TEM8 and cell lines transfected with TEM8-encoding DNA that do not normally express TEM8, such as the HCT116 cell line.

It will be appreciated that any of the above assays may be performed using the antibody-drug conjugates of the present invention in place of or in addition to anti-TEM8 antibodies.

It will be appreciated that any of the above assays may be performed using a combination of anti-TEM8 antibodies and other active agents.

In an eighth aspect of the present invention, there is provided a composition, such as a pharmaceutical composition or pharmaceutical formulation, comprising an antibody or antigen-binding region fragment thereof or an antibody-drug conjugate thereof of the present invention.

The term "pharmaceutical composition" as used herein refers to a composition that is present in a form that allows the biological activity of the active ingredients contained therein to be effective, and which does not contain additional ingredients having unacceptable toxicity to the subject to which the composition is administered.

In some embodiments, the pharmaceutical composition or pharmaceutical formulation further comprises a pharmaceutically acceptable auxiliary material, such as a pharmaceutically acceptable carrier, a pharmaceutically acceptable excipient, including buffers, as known in the art. As used herein, "pharmaceutically acceptable carrier" includes any and all solvents, dispersion media, isotonic agents, and absorption delaying agents, and the like that are physiologically compatible.

See also "Handbook of Pharmaceutical Excipients", 8th Edition, R.C.Rowe, P.J.Seskey, and S.C. Owen, Pharmaceutical Press, London, Chicago, for the use of pharmaceutically acceptable auxiliary materials and uses thereof.

The compositions or formulation of the present invention may be in a variety of forms. Such forms include, for example, liquid, semi-solid, and solid dosage forms, such as liquid solutions (e.g., injectable and infusible solutions), powders or suspensions, liposomal formulations, and suppositories. The preferred form depends on the intended mode of administration and therapeutic use.

Pharmaceutical formulation comprising the antibody of the present invention as described herein may be prepared by mixing the antibody of the present invention having the desired purity with one or more pharmaceutically acceptable auxiliary materials, preferably in the form of lyophilized formulations or aqueous solutions.

The pharmaceutical compositions or formulations of the present invention may also contain more than one active ingredient as required for the particular indication being treated, preferably those having complementary activities that do not adversely affect each other. For example, it is desirable to also provide additional therapeutic agents, for example, chemotherapeutic agents, cytokines, cytotoxic agents, other antibodies, small molecule drugs, or immunomodulators (e.g., immunosuppressants). The active ingredients are suitably present in combination in an amount effective for the intended use.

In a ninth aspect of the present invention, there is provided a pharmaceutical combination or a pharmaceutical combination product comprising an anti-TEM8 antibody or fragment thereof (preferably an antigen-binding fragment) of the present invention, or an antibody-drug conjugate thereof, and one or more additional therapeutic agents (e.g., such as chemotherapeutic agents, cytokines, cytotoxic agents, other antibodies, vaccines, small molecule drugs, or immunomodulators (e.g., immunosuppressants).

The term "pharmaceutical combination or combination product" as used herein refers to a non-fixed combination product or a fixed combination product, including but not limited to a kit of parts/a kit. The term "non-fixed combination" means that the active ingredients (e.g., (i) an antibody or antibody-drug conjugate thereof of the present invention, and (ii) additional therapeutic agents) are administered to a patient as separate entities either simultaneously, without specific time limitations, or sequentially at the same or different time intervals, wherein such administration provides prophylactically or therapeutically effective levels of the two or more active agents in the patient. The term "fixed combination" means that two or more active agents are administered to a patient simultaneously in the form of a single entity. The dosage and/or time intervals of the two or more active agents are preferably selected such that the combined use of the components results in a greater effect in the treatment of the disease or disorder than that would be achieved by use of either component alone. The ingredients may each be in separate formulations, which may be the same or different.

In a tenth aspect of the present invention, there is provided a kit of parts comprising the pharmaceutical combination of the present invention, preferably in the form of pharmaceutical dosage units. Dosage units may thus be provided according to a dosing regimen or interval of drug administration.

In one embodiment, a kit of parts according to the present invention comprises, in the same package:
- a first container containing a pharmaceutical composition comprising an anti-TEM8 antibody or antibody-drug conjugate thereof;
- a second container containing a pharmaceutical composition comprising additional therapeutic agents such as chemotherapeutic agents, cytokines, cytotoxic agents, other antibodies, vaccines, small molecule drugs, or immunomodulators (e.g., immunosuppressants).

In an eleventh aspect of the invention, there is provided a method for preventing or treating TEM8-associated diseases in a subject, comprising administering to the subject an effective amount (e.g., a preventive or therapeutically effective amount) of an anti-TEM8 antibody or antigen-binding fragment thereof, an antibody-drug conjugate, a pharmaceutical composition, or a combination product of the present invention.

As used herein, the term "treating" (or "treat" or "treatment") refers to slowing, interrupting, arresting, relieving, stopping, lowering, or reversing the onset of the symptoms, complications, or biochemical indicators of a disease, relieving the symptoms, or preventing or inhibiting the further development of the disease, condition, or disorder.

As used herein, "preventing" (or "prevent" or "prevention") includes the inhibition of the development or progression of symptoms of a disease or disorder, or a specific disease or disorder.

The term "effective amount" as used herein refers to an amount or dose of an antibody or fragment or composition or combination of the present invention which, upon administration to a patient in a single or multiple dose, produces the desired effect in the patient in need of treatment or prevention.

A "therapeutically effective amount" as used herein refers to an amount effective, at required dosages and for periods of time required, to achieve the desired therapeutic result. A therapeutically effective amount is also one in which any toxic or adverse effect of the antibody or antibody fragment or composition or combination is less than a therapeutically beneficial effect. A "therapeutically effective amount" preferably inhibits a measurable parameter (e.g., tumor volume) or improve a measurable parameter by at least about 40%, even more preferably at least about 50%, 55%, 60%, 65%, 70%, 75%, 80%, 85%, 90% or even 100% relative to untreated subjects.

A "prophylactically effective amount" as used herein refers to an amount effective, at required dosages and for periods of time required, to achieve the desired prophylactic result. Generally, since a prophylactic dose is administered in a subject before or at an earlier stage of a disease, the prophylactically effective amount will be less than the therapeutically effective amount.

An "individual", or "subject" includes mammals. Mammals include, but are not limited to, domestic animals (e.g., cows, sheep, cats, dogs, and horses), primates (e.g., humans and non-human primates such as monkeys), rabbits, and rodents (e.g., mice and rats). In some embodiments, the individual or subject is human.

In some embodiments, a TEM8-associated disease described herein is a disease associated with abnormal growth and/or dysfunction of tumor stromal cells, e.g., a tumor, e.g., a cancer.

In some embodiments, the TEM8-associated disease described herein includes a tumor, e.g., a cancer. The cancer may be in an early, intermediate, or advanced stage or may be metastatic. In some embodiments, the cancer may be a solid tumor or a hematological tumor. In some embodiments, the cancer is selected from colon cancer, rectal cancer, and colorectal cancer.

In one embodiment, the tumor refers to a tumor in the tumor tissue or tumor cells of the individual that, for example, has TEM8 expression or has a TEM8 nucleic acid, or an elevated level of TEM8 protein (e.g., expression) or an elevated nucleic acid level of TEM8, as compared to an adjacent normal tissue or normal cells (e.g., normal cells in the tissue) of the individual or the same tissue or cells therein of a healthy individual.

In some embodiments, an antibody or antibody fragment or antibody-drug conjugate or composition or formulation or combination product of the present invention delays the onset of a disorder and/or symptoms associated with the disorder.

In some embodiments, the methods of prevention or treatment described herein further comprise administering to the subject or individual a combination of an antibody molecule or pharmaceutical composition or antibody-drug conjugate disclosed herein, and one or more additional therapies, e.g., treatment modalities and/or additional therapeutic agents.

In some embodiments, the treatment modalities include surgery, radiation therapy (e.g., an external particle beam therapy, which involves three-dimensional conformal radiation therapy in which an irradiation area is designed), localized irradiation (e.g., irradiation directed at a preselected target or organ), or focused irradiation, and the like.

In some embodiments, the therapeutic agents are selected from chemotherapeutic agents, cytokines, cytotoxic agents, other antibodies, vaccines, small molecule drugs, or immunomodulators (e.g., immunosuppressants).

In some embodiments, the antibodies herein can be administered in combination with other antibodies, either separately, for example, as individual antibodies administered independently; or conjointly (e.g., as bispecific or multispecific antibody molecules).

The combination therapies of the present invention encompass both administration in combination (e.g., two or more therapeutic agents contained in the same preparation or separate preparations), and separate administration, in which case administration of the antibody of the present invention, as well as antibody-drug conjugates, compositions, pharmaceutical compositions, formulations, etc., comprising the same, may occur prior to, concurrently with, and/or after administration of the additional therapeutic agents and/or agents.

The anti-TEM8 antibody of the present invention (as well as an antibody-drug conjugate, composition, pharmaceutical composition, formulation, combination product, etc. comprising the same) may be administered by any suitable method, including parenteral administration, and intralesional administration if local treatment is required. Parenteral injection or infusion includes intramuscular, intravenous, intra-arterial, intraperitoneal, or subcutaneous injection or infusion. Administration may be by any suitable route, e.g., by injection, e.g., intravenous or subcutaneous, depending on whether administration is short-term or long-term to some extent. Various administration schedules are encompassed herein, including, but not limited to, single administration or multiple administration at multiple time points, bolus administration, and pulse infusion.

In other aspects, the present invention provides the use of the anti-TEM8 antibody or fragment thereof of the present invention, or an antibody-drug conjugate or composition comprising the same, in the manufacture or preparation of a medicament for use as described herein, for example, for the prevention or treatment of a related disease or disorder mentioned herein.

In a twelfth aspect of the present invention, the present invention also relates to a method for the diagnosis and detection of the antibody or antigen-binding fragment thereof of the present invention, and a composition comprising the same for the diagnosis and detection.

In certain embodiments, any anti-TEM8 antibody or antigen-binding fragment thereof provided herein may be used to detect the presence of TEM8 in a biological sample.

The term "detect" as used herein includes quantitative or qualitative detection; exemplary detection methods may involve immunohistochemistry, immunocytochemistry, flow cytometry (e.g., FACS), antibody molecule complexed magnetic beads, ELISA assays, and PCR-technology (e.g., RT-PCR). In certain embodiments, the biological sample is blood, serum, or other liquid sample of biological origin. In certain embodiments, the biological sample comprises cells or tissue. In certain embodiments, the biological sample is from a tumor or cancer tissue.

In one embodiment, provided is an anti-TEM8 antibody or antigen-binding fragment thereof for the diagnosis and detection method.

In another aspect, provided is a method of detecting the presence of TEM8 in a biological sample. In certain embodiments, the method comprises detecting the presence of TEM8 in a biological sample. In certain embodiments, TEM8 is human TEM8 or mouse, rat, dog, or cynomolgus TEM8. In certain embodiments, the method comprises contacting a biological sample with an anti-TEM8 antibody as described herein under conditions that permit the binding of the anti-TEM8 antibody to TEM8, and detecting whether a complex is formed between the anti-TEM8 antibody and TEM8. The formation of complex indicates the presence of TEM8. The method may be an in vitro or in vivo method. In one embodiment, the anti-TEM8 antibody is used to select subjects suitable for treatment with the anti-TEM8 antibody, e.g., wherein TEM8 is a biomarker used to select the subject.

In certain embodiments, there is provided a labeled antibody or fragment thereof. The labels include, but are not limited to, labels or moieties that are detected directly (e.g., fluorescent labels, chromophore labels, electron-dense labels, chemiluminescent labels, and radioactive labels), as well as moieties that are detected indirectly, such as enzymes or ligands, for example, by enzymatic reactions or molecular interactions.

In some embodiments provided herein, the sample is obtained prior to treatment with an antibody or fragment thereof of the present invention. In some embodiments, the sample is obtained prior to treatment with additional therapies. In some embodiments, the sample is obtained during or after the treatment with the additional therapies.

In some embodiments, TEM8 is detected prior to treatment, e.g., prior to initiation of treatment or prior to a certain treatment after a treatment interval.

In some embodiments, there is provided a method for treating TEM8-associated diseases of the present invention, the method comprising: testing a subject (e.g., a sample) (e.g., a subject sample) for the presence of TEM8, thereby determining a TEM8 value; comparing the TEM8 value with a control value (e.g., a value in the same tissue or cells of a healthy individual or in adjacent normal tissue or cells of a patient to be tested); and administering to the subject a therapeutically effective amount of an antibody or fragment thereof, or an antibody-drug conjugate, composition, pharmaceutical composition, formulation, or combination product comprising the same, optionally in combination with one or more additional therapies, if the TEM8 value is greater than the control value, thereby treating the disease.

In a thirteenth aspect, the present invention relates to the specific embodiments as follows:
1. An antibody specifically binding to TEM8 or an antigen-binding fragment thereof, wherein the antibody comprises:
1) 3 complementarity-determining regions HCDR1, HCDR2, and HCDR3 contained in VH as set forth in SEQ ID NO: 1, and 3 complementarity-determining regions LCDR1, LCDR2, and LCDR3 contained in VL as set forth in SEQ ID NO: 2;
2) 3 complementarity-determining regions HCDR1, HCDR2, and HCDR3 contained in VH as set forth in SEQ ID NO: 14, and 3 complementarity-determining regions LCDR1, LCDR2, and LCDR3 contained in VL as set forth in SEQ ID NO: 15;
3) 3 complementarity-determining regions HCDR1, HCDR2, and HCDR3 contained in VH as set forth in SEQ ID NO: 27, and 3 complementarity-determining regions LCDR1, LCDR2, and LCDR3 contained in VL as set forth in SEQ ID NO: 28 or 40;
4) 3 complementarity-determining regions HCDR1, HCDR2, and HCDR3 contained in VH as set forth in SEQ ID NO: 42, and 3 complementarity-determining regions LCDR1, LCDR2, and LCDR3 contained in VL as set forth in SEQ ID NO: 43; or
5) 3 complementarity-determining regions HCDR1, HCDR2, and HCDR3 contained in VH as set forth in SEQ ID NO: 55, and 3 complementarity-determining regions LCDR1, LCDR2, and LCDR3 contained in VL as set forth in SEQ ID NO: 56;
preferably, wherein the HCDR and the LCDR are determined according to the IMGT or Kabat scheme.
2. An antibody specifically binding to TEM8 or antigen-binding fragment thereof, wherein the antibody comprises: 3 complementarity-determining regions HCDR1, HCDR2 and HCDR3 of the heavy chain variable region and 3 complementarity-determining regions LCDR1, LCDR2 and LCDR3 of the light chain variable region, wherein the HCDR1, HCDR2, HCDR3, LCDR1, LCDR2, and LCDR3 are based on the IMGT scheme, comprising or consisting of, respectively, the amino acid sequence as set forth in SEQ ID NOs in the table below:

| HCDR1 (IMGT) comprising or consisting of the SEQ ID NOs below: | HCDR2 (IMGT) comprising or consisting of the SEQ ID NOs below: | HCDR3 (IMGT) comprising or consisting of the SEQ ID NOs below: | LCDR1 (IMGT) comprising or consisting of the SEQ ID NOs below: | LCDR2 (IMGT) comprising or consisting of the SEQ ID NOs below: | LCDR3 (IMGT) comprising or consisting of the SEQ ID NOs below: |
|---|---|---|---|---|---|
| 3 | 4 | 5 | 6 | 7 | 8 |
| 16 | 17 | 18 | 19 | 20 | 21 |
| 29 | 30 | 31 | 32 | 33 | 34 |
| 29 | 30 | 31 | 32 | 33 | 41 |
| 44 | 45 | 46 | 47 | 48 | 49 |
| 57 | 58 | 59 | 60 | 61 | 62 |

or wherein the HCDR1, HCDR2, HCDR3, LCDR1, LCDR2, and LCDR3 are based on the Kabat scheme, comprising or consisting of, respectively, the amino acid sequence as set forth in SEQ ID NOs in the table below:

| HCDR1 (Kabat) comprising or consisting of the SEQ ID NOs below: | HCDR2 (Kabat) comprising or consisting of the SEQ ID NOs below: | HCDR3 (Kabat) comprising or consisting of the SEQ ID NOs below: | LCDR1 (Kabat) comprising or consisting of the SEQ ID NOs below: | LCDR2 (Kabat) comprising or consisting of the SEQ ID NOs below: | LCDR3 (Kabat) comprising or consisting of the SEQ ID NOs below: |
|---|---|---|---|---|---|
| 9 | 10 | 11 | 12 | 13 | 8 |
| 22 | 23 | 24 | 25 | 26 | 21 |
| 35 | 36 | 37 | 38 | 39 | 34 |
| 35 | 36 | 37 | 38 | 39 | 41 |
| 50 | 51 | 52 | 53 | 54 | 49 |
| 63 | 64 | 65 | 66 | 67 | 62 |

3. The TEM8-binding antibody or antigen-binding fragment thereof as described in embodiment 1 or 2, wherein the antibody comprises a heavy chain variable region, wherein the heavy chain variable region comprises or consists an amino acid sequence having at least 90%, 91%, 92%, 93%, 94%, 95%, 96%, 97%, 98%, or 99% identity to the amino acid sequence selected from the group consisting of SEQ ID NO: 1, 14, 27, 42, or 55; or the heavy chain variable region comprises or consists of the amino acid sequence selected from the group consisting of SEQ ID NO: 1, 14, 27, 42, or 55; and/or
the antibody comprises a light chain variable region, wherein the light chain variable region comprises or consists an amino acid sequence having at least 90%, 91%, 92%, 93%, 94%, 95%, 96%, 97%, 98%, or 99% identity to the amino acid sequence selected from the group consisting of SEQ ID NO: 2, 15, 28, 40, 43, or 56; or the light chain variable region comprises or consists of the amino acid sequence selected from the group consisting of SEQ ID NO: 2, 15, 28, 40, 43, or 56.
4. An anti-TEM8 antibody or antigen-binding fragment thereof, wherein the antibody comprises a heavy chain variable region and a light chain variable region, wherein
(i) the heavy chain variable region comprises an amino acid sequence having at least 90%, 91%, 92%, 93%, 94%, 95%, 96%, 97%, 98%, or 99% identity to the amino acid sequence as set forth in SEQ ID NO: 1, and the light chain variable region comprises an amino acid sequence having at least 90%, 91%, 92%, 93%, 94%, 95%, 96%, 97%, 98%, or 99% identity to the amino acid sequence as set forth in SEQ ID NO: 2;
(ii) the heavy chain variable region comprises an amino acid sequence having at least 90%, 91%, 92%, 93%, 94%, 95%, 96%, 97%, 98%, or 99% identity to the amino acid sequence as set forth in SEQ ID NO: 14, and the light chain variable region comprises an amino acid sequence having at least 90%, 91%, 92%, 93%, 94%, 95%, 96%, 97%, 98%, or 99% identity to the amino acid sequence as set forth in SEQ ID NO: 15;
(iii) the heavy chain variable region comprises an amino acid sequence having at least 90%, 91%, 92%, 93%, 94%, 95%, 96%, 97%, 98%, or 99% identity to the amino acid sequence as set forth in SEQ ID NO: 27, and the light chain variable region comprises an amino acid sequence having at least 90%, 91%, 92%, 93%, 94%, 95%, 96%, 97%, 98%, or 99% identity to the amino acid sequence as set forth in SEQ ID NO: 28 or 40;
(iv) the heavy chain variable region comprises an amino acid sequence having at least 90%, 91%, 92%, 93%, 94%, 95%, 96%, 97%, 98%, or 99% identity to the amino acid sequence as set forth in SEQ ID NO: 42, and the light chain variable region comprises an amino acid sequence having at least 90%, 91%, 92%, 93%, 94%, 95%, 96%, 97%, 98%, or 99% identity to the amino acid sequence as set forth in SEQ ID NO: 43;
(v) the heavy chain variable region comprises an amino acid sequence having at least 90%, 91%, 92%, 93%, 94%, 95%, 96%, 97%, 98%, or 99% identity to the amino acid sequence as set forth in SEQ ID NO: 55, and the light chain variable region comprises an amino acid sequence having at least 90%, 91%, 92%, 93%, 94%, 95%, 96%, 97%, 98%, or 99% identity to the amino acid sequence as set forth in SEQ ID NO: 56.

5. The TEM8-binding antibody or antigen-binding fragment thereof according to embodiment 1 or 2, wherein the antibody comprises a heavy chain variable region and a light chain variable region, wherein the heavy chain variable region and the light chain variable region comprise or consist of, respectively, the amino acid sequences as set forth in SEQ ID Nos in the table below:

| VH | VL |
|---|---|
| SEQ ID NO: 1 | SEQ ID NO: 2 |
| SEQ ID NO: 14 | SEQ ID NO: 15 |
| SEQ ID NO: 27 | SEQ ID NO: 28 |
| SEQ ID NO: 27 | SEQ ID NO: 40 |
| SEQ ID NO: 42 | SEQ ID NO: 43 |
| SEQ ID NO: 55 | SEQ ID NO: 56 |

6. The TEM8-binding antibody or antigen-binding fragment thereof according to any one of embodiments 1-5, wherein the antibody comprises a heavy chain constant region, for example, a heavy chain constant region of IgG1, IgG2, IgG3, or IgG4, e.g., a heavy chain constant region of IgG1, which
(i) comprises an amino acid sequence having at least 85%, 90%, 91%, 92%, 93%, 94%, 95%, 96%, 97%, 98%, or 99% identity to the amino acid sequence selected from the group consisting of SEQ ID NO: 71; or
(ii) comprises or consists of the amino acid sequence selected from the group consisting of SEQ ID NO: 71.

7. The TEM8-binding antibody or antigen-binding fragment thereof according to any one of embodiments 1-6, wherein the antibody comprises a light chain constant region, for example, a lambda or kappa light chain constant region, e.g., the light chain constant region
(i) comprises or consists of an amino acid sequence having at least 85%, 90%, 91%, 92%, 93%, 94%, 95%, 96%, 97%, 98%, or 99% identity to the amino acid sequence selected from the group consisting of SEQ ID NO: 72 or 73; or
(ii) comprises or consists of an amino acid sequence selected from the group consisting of SEQ ID NO: 72 or 73.

8. The TEM8-binding antibody or antigen-binding fragment thereof according to any one of embodiments 1-7, wherein the antibody comprises a heavy chain constant region and a light chain constant region, wherein
the heavy chain constant region comprises or consists of the amino acid sequence as set forth in SEQ ID NO: 71, and the light chain constant region comprises or consists of the amino acid sequence as set forth in SEQ ID NO: 72 or 73.
9. The TEM8-binding antibody or antigen-binding fragment thereof according to any one of embodiments 1-8, wherein the antibody comprises a heavy chain and a light chain, wherein the heavy chain comprises a heavy chain variable region and a heavy chain constant region, and the light chain comprises a light chain variable region and a light chain constant region, wherein
(i). the heavy chain variable region comprises or consists of the amino acid sequence as set forth in SEQ ID NO:1, the light chain variable region comprises or consists of the amino acid sequence as set forth in SEQ ID NO:2, the heavy chain constant region comprises or consists of the amino acid sequence as set forth in SEQ ID NO:71, and the light chain constant region comprises or consists of the amino acid sequence as set forth in SEQ ID NO: 72;
(ii). the heavy chain variable region comprises or consists of the amino acid sequence as set forth in SEQ ID NO: 14, the light chain variable region comprises or consists of the amino acid sequence as set forth in SEQ ID NO: 15, the heavy chain constant region comprises or consists of the amino acid sequence as set forth in SEQ ID NO:71, and the light chain constant region comprises or consists of the amino acid sequence as set forth in SEQ ID NO: 72;
(iii). the heavy chain variable region comprises or consists of the amino acid sequence as set forth in SEQ ID NO: 27, the light chain variable region comprises or consists of the amino acid sequence as set forth in SEQ ID NO: 28 or 40, the heavy chain constant region comprises or consists of the amino acid sequence as set forth in SEQ ID NO: 71, and the light chain constant region comprises or consists of the amino acid sequence as set forth in SEQ ID NO: 73;
(iv). the heavy chain variable region comprises or consists of the amino acid sequence as set forth in SEQ ID NO:42, the light chain variable region comprises or consists of the amino acid sequence as set forth in SEQ ID NO: 43, the heavy chain constant region comprises or consists of the amino acid sequence as set forth in SEQ ID NO:71, and the light chain constant region comprises or consists of the amino acid sequence as set forth in SEQ ID NO: 73; or
(v). the heavy chain variable region comprises or consists of the amino acid sequence as set forth in SEQ ID NO:55, the light chain variable region comprises or consists of the amino acid sequence as set forth in SEQ ID NO:56, the heavy chain constant region comprises or consists of the amino acid sequence as set forth in SEQ ID NO:71, and the light chain constant region comprises or consists of the amino acid sequence as set forth in SEQ ID NO: 73.

10. The TEM8-binding antibody or antigen-binding fragment thereof according to any one of embodiments 1-8, having one or more of the following properties:
(1) being capable of binding with high affinity to TEM8, e.g., to TEM8 of human, cynomolgus monkey, rat, or mouse;
(2) being capable of binding with high affinity to TEM8 expressed on the cell membrane surface, e.g., to TEM8 of human, cynomolgus monkey, rat, or mouse;
(3) being capable of inducing endocytosis of the antibody or fragment thereof or a molecule comprising the antibody or fragment thereof (e.g., an ADC) by a TEM8-positive cell (e.g., a TEM8-overexpressing CHO cell);
(4) being capable of targeting and killing a TEM8-positive cells, such as a TEM8-positive tumor cell;
(5) being capable of treating a tumor, such as a cancer;
(6) being suitable for constructing an antibody-drug conjugate (ADC) for targeted killing of TEM8-positive cells, such as TEM8-positive tumor cells, or for the treatment of tumors such as cancer;
(7) inhibiting the binding of any of the antibodies listed in embodiment 9 to TEM8;
(8) binding to the same or an overlapping epitope as any of the antibodies of embodiment 9; or
(9) competing for binding to TEM8 with any of the antibodies of embodiment 9.

11. The antibody or antigen-binding fragment thereof according to any one of the embodiments 1-10, wherein the antibody is a monoclonal antibody.
12. The antibody or antigen-binding fragment thereof according to any one of the embodiments 1-11, wherein the antibody is a human antibody.
13. The antibody or antigen-binding fragment thereof according to any one of embodiments 1-12, wherein the antigen-binding fragment is an antibody fragment selected from the group consisting of Fab, Fab', Fab'-SH, Fv, a single chain antibody such as scfv, a (Fab')2 fragment, a single domain antibody, a diabody, or a linear antibody.
14. The antibody or antigen-binding fragment thereof according to embodiment 13, wherein the single-chain antibody is scFv.
15. The antibody or antigen-binding fragment thereof according to any one of the embodiments 1-14, wherein the antibody is a bispecific or multispecific antibody.
16. An isolated nucleic acid encoding the anti-TEM8 antibody or antigen-binding fragment thereof of any one of the embodiments 1-15.
17. An expression vector comprising the nucleic acid of embodiment 16.
18. The expression vector according to embodiment 17, wherein the expression vector is a pCDNA vector, e.g., a pCDNA3.4 vector.
19. A host cell comprising the nucleic acid of embodiment 16 or the expression vector of embodiment 17 or 18.
20. The host cell according to embodiment 19, wherein the host cell is prokaryotic or eukaryotic.
21. The host cell according to embodiment 20, wherein the host cell is selected from the group consisting of Escherichia coli cells, yeast cells, mammalian cells, or other cells suitable for preparing an antibody or antigen-binding fragment thereof.
22. The host cell according to embodiment 21, wherein the host cell is a 293 cell or a CHO cell.
23. A method of preparing an anti-TEM8 antibody or antigen-binding fragment thereof, comprising culturing the host cell of any one of embodiments 19-22 under conditions suitable for expression of the nucleic acid encoding the anti-TEM8 antibody or antigen-binding fragment thereof of any one of embodiments 1-15, optionally isolating the antibody or antigen-binding fragment thereof, optionally the method further comprising recovering the anti-TEM8 antibody or antigen-binding fragment thereof from the host cell.
24. An antibody-drug conjugate comprising the antibody or antigen-binding fragment thereof of any one of the embodiments 1-15 and additional therapeutic agents.
25. The antibody-drug conjugate according to embodiment 24, wherein the additional therapeutic agent is a cytotoxic agent, e.g., an MMAE, optionally the MMAE conjugates to an antibody or antigen-binding fragment thereof via a Vc linker, e.g., the antibody or antigen-binding fragment thereof is linked to VcMMAE to form an antibody-drug conjugate.
26. A pharmaceutical composition comprising the antibody or antigen-binding fragment thereof of any one of embodiments 1-15 or the antibody-drug conjugate of embodiment 24 or 25, and optionally a pharmaceutically acceptable auxiliary material.
27. A pharmaceutical composition comprising the antibody or antigen-binding fragment thereof of any one of embodiments 1-15 or the antibody-drug conjugate of embodiment 24 or 25, and additional therapeutic agents.
28. A pharmaceutical combination comprising the antibody or antigen-binding fragment thereof according to any one of embodiments 1-15 or the antibody-drug conjugate of embodiment 24 or 25, and additional therapeutic agents.
29. The pharmaceutical composition according to embodiment 27 or the pharmaceutical combination of embodiment 28, wherein the additional therapeutic agents are selected from the group consisting of chemotherapeutic agents, cytokines, cytotoxic agents, other antibodies, small molecule drugs, or immunomodulators (e.g., immunosuppressants).
30. Use of an effective amount of the anti-TEM8 antibody or antigen-binding fragment thereof of any one of embodiments 1-15, or the antibody-drug conjugate of embodiment 24 or 25, or the pharmaceutical composition of embodiment 26, in the preparation of a medicament for preventing or treating a tumor in a subject or individual.
31. The use according to embodiment 30, wherein the tumor is a solid tumor, e.g., the tumor is a cancer, e.g., the cancer is selected from the group consisting of colon cancer, rectal cancer, or colorectal cancer.
32. The use of embodiment 30 or 31, further comprising administering to the subject one or more additional therapies in combination.
33. The use according to embodiment 32, wherein the therapy comprises treatment modalities and/or additional therapeutic agents.
34. The use according to embodiment 33, wherein the additional therapeutic agents are selected from the group consisting of chemotherapeutic agents, cytokines, other antibodies, cytotoxic agents, vaccines, small molecule drugs, or immunomodulators (e.g., immunosuppressants), and/or the treatment modalities comprise surgical treatment and/or radiation therapy.
35. A method of detecting TEM8 in a sample comprising
(a) contacting the sample with the anti-TEM8 antibody or antigen-binding fragment thereof of any of the embodiments 1-15; and
(b) detecting the formation of a complex between an anti-TEM8 antibody or antigen-binding fragment thereof and TEM8; optionally, the anti-TEM8 antibody is detectably labeled.

All publications, patent applications, patents, and other references mentioned herein are incorporated by reference in their entirety. Any or all of the features discussed above and throughout this application may be combined in various embodiments of the present invention. In addition, the materials, methods, and examples described herein are illustrative only and not intended to be limiting. Other features, objects, and advantages of the present invention will be apparent from the description and drawings, and from the appended claims.

### Example:

### Example 1. TEM8 antigen species sequence homology and construction of stable high-expression cell lines

Homology analysis was performed on different species of TEM8 sequences (human TEM8 (hTEM8, SEQ ID NO: 68), cynomolgus monkey TEM8 (CynotTEM8), mouse TEM8 (mTEM8, SEQ ID NO: 69), rat TEM8 (ratTEM8), and dog TEM8 (dogTEM8, SEQ ID NO: 70)), and TEM8 was found to be highly homologous in human, cynomolgus monkey, mouse, and rat. The extracellular region sequences of human TEM8 and cynomolgus monkey TEM8 are identical. The sequence of mouse TEM8 and rat TEM8 are identical. The sequence homology between human TEM8 and mouse TEM8 is 98.9% (see Table 1).

**Table 1: Homology of TEM8 sequences in different species**

| Seq-> | hTEM8 | CynoTEM8 | mTEM8 | ratTEM8 | dogTEM8 |
|---|---|---|---|---|---|
| hTEM8 | ID | 1.000 | 0.989 | 0.989 | 0.979 |
| CynoTEM8 | 1.000 | ID | 0.989 | 0.989 | 0.979 |
| mTEM8 | 0.989 | 0.989 | ID | 1.000 | 0.979 |
| ratTEM8 | 0.989 | 0.989 | 1.000 | ID | 0.979 |
| dogTEM8 | 0.979 | 0.979 | 0.979 | 0.979 | ID |

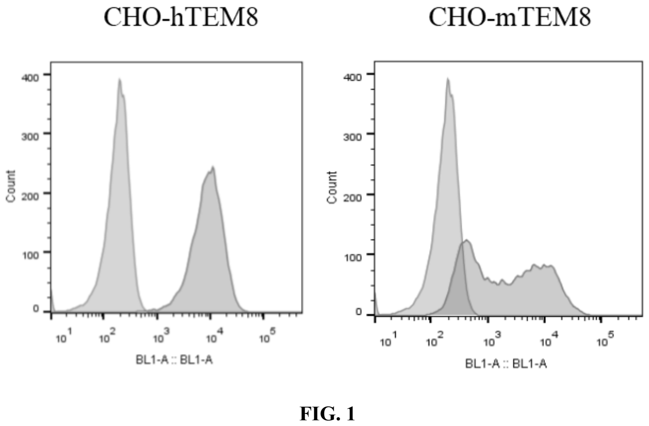

The nucleic acids encoding human TEM8(SEQ ID NO:68) and mouse TEM8(SEQ ID NO:69) were cloned into expression vector pCDNA3.1(+) (constructed by Beijing Tsingke Biotech Co., Ltd.), respectively, which was transfected into CHO-K1 cells (Cobioer Biosciences Co., Ltd., Cat: CBP60296) with transfection reagent Lipofectamine^{™} 3000(ThermoFisher, L3000075), and the cells were subjected to flow cell sorting 14 days after transfection, with the
primary antibody being L2 (67 nM) (L2 antibody heavy chain: SEQ ID NO: 74; L2 antibody light chain: SEQ ID NO: 75) and the second antibody being goat anti-human IgG H & L (FITC) (abcam, 6854), and cells with a positive rate of 2.5% were selected for culture, and flow cytometry analysis was performed before the stable cell line culture was completed. Among them, CHO stable cells with high expression of human TEM8 were designated CHO-hTEM8, and CHO stable cells with high expression of mouse TEM8 were designated CHO-mTEM8. CHO-hTEM8 and CHO-mTEM8 were detected by flow cytometry analysis, with the primary antibody being L2 (67 nM) (L2 antibody heavy chain: SEQ ID NO: 74; L2 antibody light chain: SEQ ID NO: 75) and the secondary antibody being goat anti-human IgG H&L (FITC)(abcam, ab6854), and the results were shown in FIG. 1, wherein the left panel showed the flow cytometry results of CHO-hTEM8, and the right panel showed the flow cytometry results of CHO-mTEM. The results showed that CHO-hTEM8 and CHO-mTEM8 cells had high expression of hTEM8 and mTEM8, respectively.

### Example 2. Screening for TEM8-specific antibodies using a fully human scFv phage library

The process of screening the fully human scFv phage antibody was as follows: 2×10^12 phage library (CRO, human naive scFv library) was added to 1×10^7 CHO-K1 negative cells (Cobioer Biosciences Co., Ltd., Cat: CBP60296) and incubated for 2 h at 4°C. The phage suspension after negative panning was added to 1×10^7 CHO-hTem8 and incubated for 2h at 4°C. The above incubated mixture was washed 8-11 times with PBS and centrifuged to remove unbound phages, and the cell-bound phages were eluted with elution buffer (glycine-hydrochloric acid buffer, 0.2M, pH 2.5). After 3 rounds of the above panning, specific binding between phage supernatant and hTEM8-his antigen (hTEM8 containing 6 His tags) was detected to obtain positive clones, and the results are shown in Table 2.

**Table 2: TEM8-specific phage clones**

| Phage clone | hTEM8-his ELISA (OD450) | Blank ELISA (OD450) |
|---|---|---|
| 3A3 | 2.81 | 0.13 |
| 3B3 | 2.86 | 0.12 |
| 6A6 | 2.67 | 0.28 |
| 7E3 | 2.50 | 0.10 |
| 11B7 | 2.43 | 0.23 |

Five positive phage clones were sequenced and identified to obtain the sequence results. The light chain of 6A6 original sequence contained a glycosylation site NRS, which was modified to QRS by mutation and designated as clone number 6A6Q. The sequences of the light chain variable region and heavy chain variable region of the scFv obtained by screening were shown in the sequence listing.

### Example 3. Construction of IgG antibody and FACS detection of the binding activity of IgG antibody to TEM8-overexpressing cells

### Preparation of IgG antibody

The nucleic acids encoding the light and heavy chain variable region sequences of the antibody were respectively cloned into an expression vector pCDNA 3.4 containing nucleic acids encoding the heavy chain constant region (SEQ ID NO: 71) or the light chain constant region (SEQ ID NO: 72; SEQ ID NO: 73) (the combination shown in the table below), which was co-transferred into CHO suspension cells for expression, the cell supernatant was collected and purified by a Protein A column, and the purified antibody was detected by SDS-PAGE and HPLC-SEC for the size and purity.

| Antibody name | Heavy chain variable region | Light chain variable region | Heavy chain constant region | Light chain constant region |
|---|---|---|---|---|
| 3A3 | SEQ ID NO:1 | SEQ ID NO:2 | SEQ ID NO:71 | SEQ ID NO:72 |
| 3B3 | SEQ ID NO:14 | SEQ ID NO:15 | SEQ ID NO:71 | SEQ ID NO:72 |
| 6A6 | SEQ ID NO:27 | SEQ ID NO:28 | SEQ ID NO:71 | SEQ ID NO:73 |
| 6A6Q | SEQ ID NO:27 | SEQ ID NO:40 | SEQ ID NO:71 | SEQ ID NO:73 |
| 7E3 | SEQ ID NO:42 | SEQ ID NO:43 | SEQ ID NO:71 | SEQ ID NO:73 |
| 11B7 | SEQ ID NO:55 | SEQ ID NO:56 | SEQ ID NO:71 | SEQ ID NO:73 |

### Detection of the binding activity of IgG antibody to cells

TEM8-overexpressing cells prepared in Example 1 (CHO-hTEM8 and CHO-mTEM8) were trypsinized and added to a U-bottom 96-well plate at 1×10⁶/well in 100 µL. Each of the antibodies (3A3, 6A6Q, 7E3, 11B7, 3B3, L2 (L2 antibody heavy chain: SEQ ID NO: 74; L2 antibody light chain: SEQ ID NO: 75), and Anti-hel antibody (Anti-HEL-Human IgG1, Biointron, B117901) was then subjected to a 3-fold serial dilution with PBS, starting from 26.67nM, yielding 8 concentration gradients. 100 µL of the diluted antibody was added to the cell plate to resuspend the cells, and the plate was incubated for 1h at 4°C, then washed three times with PBS. The secondary antibody Anti-Human IgG H&L (FITC, abcam, ab6854) was diluted 1:2000 with PBS., 100 µL of the diluted secondary antibody was added to the cell plate to resuspend the cells, and the plate was incubated for 1h at 4°C, then washed three times with PBS. The cells were resuspended with 100 µL PBS, the binding signals were detected by FACS, and the data were analyzed by Graphpad.

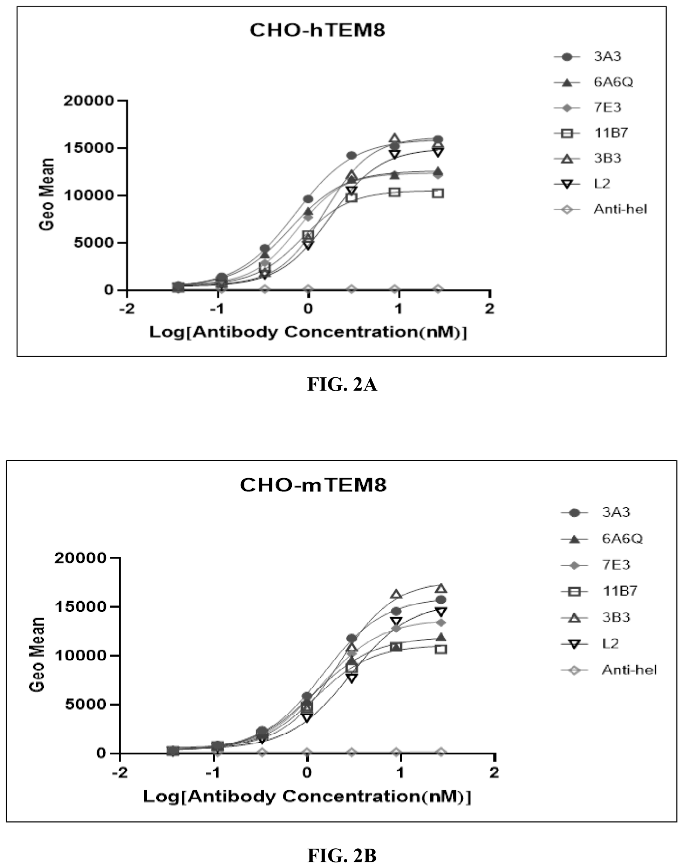

Results were shown in FIG. 2, all of five antibodies, 3A3, 6A6Q, 7E3, 11B7, 3B3, were cross-bound to cells expressing human and murine TEM8, with EC50 for binding to CHO-hTEM8 of 0.71 nM, 0.63nM, 0.75 nM, 0.85 nM, 1.51nM, respectively; EC50 for binding to CHO-mTEM8 of 1.48 nM, 1.16 nM, 1.41 nM, 1.21 nM, 2.14 nM, respectively; and wherein the control antibody L2 binds to CHO-hTEM8 and CHO-mTEM8 with EC50 of 1.72 nM, 2.84 nM, respectively.

### Example 4. Antibody endocytosis assay

The antibody endocytosis assay adopted a classical DT3C method, comprising the following steps:
TEM8-overexpressing cells (CHO-hTEM8& CHO-mTEM8) in the logarithmic growth phase were trypsinized, plated in a 96-well plate at 5000/well in 100 µL, and the plate was incubated in a 37°C, 5% CO₂ constant temperature incubator for 24 h. Recombinant DT3C protein (Recombinant DT3C protein) (CUSABIO, CSB-EP360556CQR1) was diluted to 587 nM in cell culture medium (Ham's F-12K, 10% FBS);
The antibody was diluted to 267 nM in cell culture medium. The diluted recombinant DT3C protein was mixed with the antibody in equal volume to obtain a mixture, which was incubated for 30 min at room temperature. The mixture was subjected to a 3-fold serial dilution with the cell culture medium (Ham's F-12K, 10% FBS) , yielding 10 concentration gradients. 100 µL of the diluted sample was added to the cell plate for mixing well, and the plate was incubated in a 37 °C, 5% CO₂ constant temperature incubator for 48 h. 20 µL of Cell Counting Kit-8 (CellorLab, CX001) was added to the plate, and the plate was incubated in a 37 °C, 5% CO₂ constant temperature incubator for 3 h. The absorbance value at 450 nm was measured with a microplate reader, and then the data were analyzed with Graphpad.

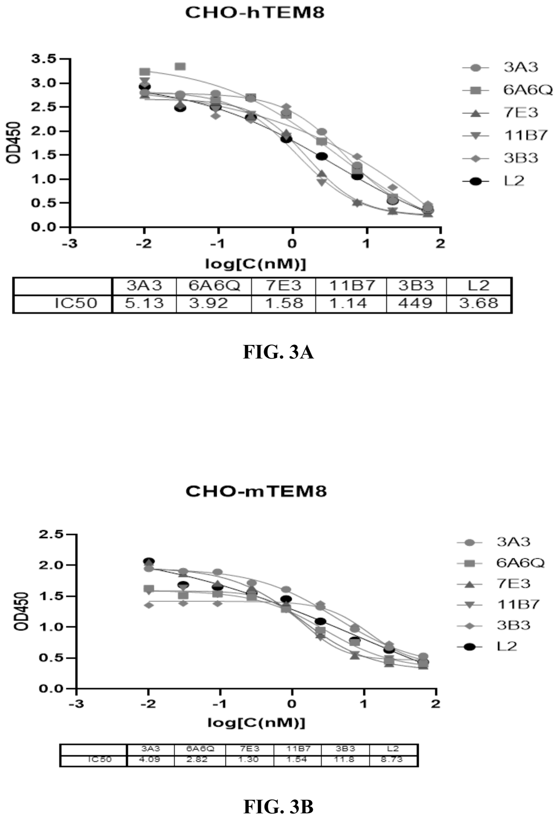

As shown in FIG. 3, the respective mixture of DT3C with five antibodies and the control antibody L2 all killed the cells, indicating that all antibodies were endocytosed by the cells.

### Example 5. Identification of antibody epitopes

The epitopes of the antibody were identified by a competitive ELISA, comprising the following steps:
The antibodies were biotinylated using EZ-Linker NHS kit (Thermo Scientific, Cat:20217). 2nM labeled antibody was mixed with 240nM unlabeled antibody at a molar ratio of 1:120, and the mixture was incubated with CHO-hTEM8 for 1h at 4°C. After incubation, the cells were washed 3 times with PBS and centrifuged to remove the supernatants. A SA-bio secondary antibody (FITC-streptavidin conjugate (AAT Bioquest, Cat: 16910) was added to the sample, and the mixture was incubated at 4°C for 1.5h, and washed 3 times with PBS. The mixture was centrifuged to remove supernatants, and the cells were resuspended in 120 µL PBS and detected by FACS for epitope binding. The results showed that 3A3, 6A6Q, 7E3, 3B3, and L2 competed for binding, belonging to the same antigenic determinant cluster Bin#1, while 11B7 did not compete for binding with all the above antibodies, belonging to another antigenic determinant cluster Bin#2, as shown in Table 3.

**Table 3: Antibody epitope**

| **Epitope Bins** | **Ab names** |
|---|---|
| Bin #1 | L2, 3A3, 6A6Q, 7E3, 3B3 |
| Bin #2 | 11B7 |

### Example 6. Preparation of TEM8 antibody-drug conjugate

Antibodies (L2, 7E3, 3A3, 6A6Q, 3B3, and 11B7) were reduced by adding an appropriate amount of a reducing agent, leaving the sulfydryl groups between the antibody chains exposed, and VcMMAE (MCE, CAS No.:646502-53-6) was dissolved in DMSO and prepared as a solution.

The dissolved VcMMAE was added according to the molar ratio of the antibody to the linker-payload of 10:1, and the antibody and VcMMAE were conjugated together via the Michael addition reaction between a sulfydryl group and a maleimide group on the Linker.

The mixture after conjugation was subjected to Buffer substitution to remove residual VcMMAE, and the purity and DAR (drug/antibody ratio) values after reaction were determined by SEC (size exclusion chromatography) and HIC (hydrophobic chromatography), respectively, and the conjugation results for different candidate molecules are shown in Table 4.

**Table 4: Preparation of TEM8 antibody-drug conjuate**

| **Sample name** (ADC) | **Purity-SEC** | **DAR** |
|---|---|---|
| L2-MMAE | 96.2% | 4.33 |
| 7E3-MMAE | 97.6% | 4.95 |
| 3A3-MMAE | 99.1% | 4.19 |
| 6A6Q-MMAE | 99.1% | 3.98 |
| 3B3-MMAE | 98.8% | 4.32 |
| 11B7-MMAE | 100.0% | 4.03 |

### Example 7. ADC in vitro cell killing assay

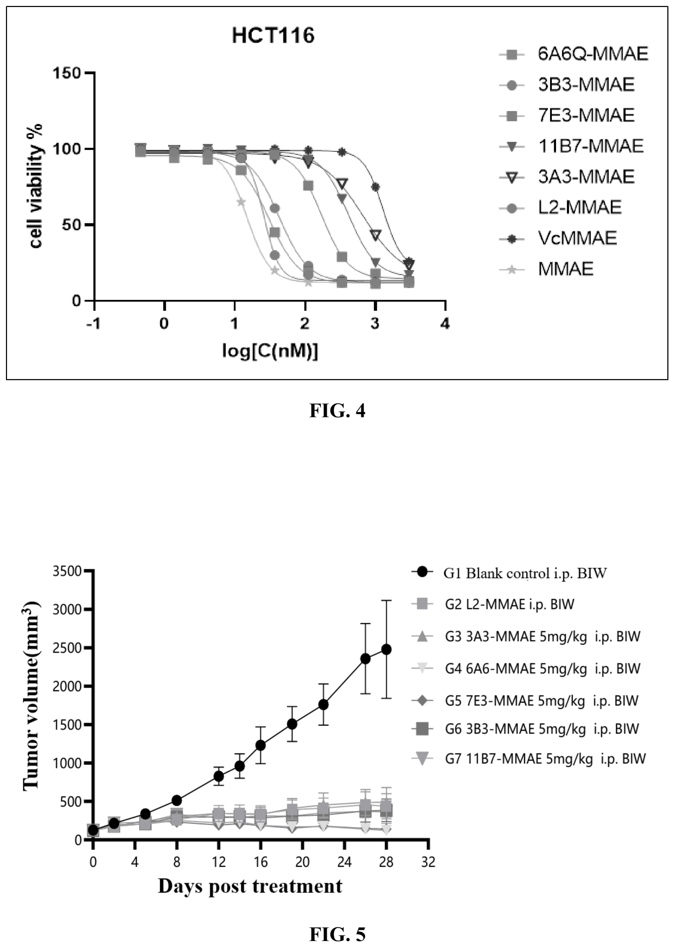

HCT116 (Cat: CBP60028, Cobioer Biosciences Co., Ltd.) in the logarithmic growth phase was trypsinized, plated in a 96-well plate at 3000 cells/well in 100 µL, and the plate was incubated in a 37°C, 5% CO2 incubator for 24 h. ADC was subjected to a 5-fold serial dilution with cell culture medium (Ham's F-12K, 10% FBS, AusGenex, Cat:FBS500-S) starting from 30000nM, yielding 10 concentration gradients. 10 µL of the diluted sample was added to the cell plate for mixing well, and the plate was incubated in a 37°C, 5% CO₂ incubator for 72 h. 10 µL of Cell Counting Kit-8 (CellorLab, CX001) was added to the plate, and the plate was incubated in a 37 °C, 5% CO₂ constant temperature incubator for 3 h. The absorbance value at 450 nm was measured with a microplate reader. Data was analyzed with Graphpad, with the in vitro killing activity of TEM8 antibody-drug conjugate shown in Table 5 and the IC50 curve shown in FIG. 4.

**Table 5: In vitro killing activity of TEM8 antibody-drug conjugate or linker + payload or payload**

| **Sample** | **IC50 (nM)** |
|---|---|
| 3B3-MMAE | 42.60 |
| 7E3-MMAE | 171.5 |
| 6A6Q-MMAE | 30.80 |
| 11B7-MMAE | 411.0 |
| 3A3-MMAE | 662.0 |
| L2-MMAE | 25.93 |
| VcMMAE | 1285 |
| MMAE | 14.76 |

### Example 8. In vivo efficacy of the HCT116 CDX model

A subcutaneous xenograft tumor model was constructed in Balb/c-nude mice (available from GemPharmatech Co., Ltd.) (Jiangsu Province Laboratory Animal Quality Certificate: 202223503) using HCT116 human colon cancer cells (Cat: CBP60028, Cobioer Biosciences Co., Ltd.), and the in vivo efficacy of each TEM8 antibody-drug conjugate was evaluated.

Specifically, HCT116 cells in the logarithmic growth phase were collected, the culture solution was removed, the cells were washed twice with PBS, and inoculated into the right dorsal side of Balb/c-nude mice at an inoculum size of 7.5*10^6/200ul/mouse (without Matrigel). After 12 days from inoculation, tumors grew to the appropriate size, mice with tumor volumes up to the standard (tumor volumes >100mm³) were randomly divided into 7 groups, with 5 animals in each group: a blank control group (PBS), a positive control L2-MMAE group, a 3A3-MMAE group, a 6A6-MMAE group, a 7E3-MMAE group, a 3B3-MMAE group, and a 11B7-MMAE group, with DPBS as vehicle for all relevant drugs.

Administration was started on the day of grouping (Day 0), with a dosing regimen of administered intraperitoneally (i.p.) at a dose of 5 mg/kg, performed twice weekly (BIW) for 28 days. Mice body weight and tumor volume were measured 3 times per week after grouping for administration. The size of the mice tumor was measured using a vernier caliper each time, and the volume was calculated using the formula V = (L × W^2)/2, where L is the longest diameter of the tumor and W is the short diameter perpendicular to the longest diameter. On day 28 after grouping for administration, i.e., after the 9th administration, the experiment was terminated and all mice were euthanized.

As shown in FIG. 5, all the dosing groups (i.e., 3A3-MMAE group, 6A6-MMAE group, 7E3-MMAE group, 3B3-MMAE group, and 11B7-MMAE group) showed significant inhibitory effect on tumor growth , with statistically significant differences compared to the blank control group.

Based on data from the entire administration cycle, the 6A6-MMAE group and the 7E3-MMAE group showed more significant advantages, both of which also showed statistically significant differences compared with the positive control L2-MMAE group (p=0.0107 and p=0.0163).

The final data showed that the tumor volume in the blank control group was 2478.9±637.3mm³; the tumor volume in the positive control L2-MMAE group was 437.6±163.5mm³; the tumor volumes in the 3A3-MMAE, 6A6-MMAE, 7E3-MMAE, 3B3-MMAE, and 11B7-MMAE groups were 487.9±192.3mm³, 123.1±17.7mm³, 141.9±51.9mm³, 383.8±147.2mm³, and 361.4±146.2mm³, respectively. The above results demonstrated that all ADC drugs had strong tumor-inhibiting activity.

### Example 9. In vivo efficacy of the HCT116 CDX model

A subcutaneous xenograft tumor model was constructed in Balb/c-nude mice (available from Shanghai Lingchang Biotechnology Co., Ltd.) (License No.: SCXK (Shanghai) 2018-0003) using HCT116 human colon cancer cells (Cobioer Biosciences Co., Ltd., Cat: CBP60028), and the in vivo efficacy of the TEM8 antibody drug 6A6Q naked antibody was evaluated.

Specifically, HCT116 cells in the logarithmic growth phase were collected, the culture solution was removed, the cells were washed twice with PBS, and inoculated into the right dorsal side of Balb/c-nude mice at an inoculum size of 1*10^7/200ul/mouse (without Matrigel). After 7 days from inoculation, tumors grew to the appropriate size, mice with tumor volumes up to the standard (tumor volumes >100mm³) were randomly divided into 2 groups, with 6 animals in each group: a blank control group (PBS) and a naked 6A6Q antibody group, respectively, with DPBS as vehicle for all relative drugs.

Administration was started on the day of grouping (Day 0), with a dosing regimen of administered intraperitoneally (i.p.) at a dose of 20mg/kg, performed once weekly (QW) for 23 days.

Mice body weight and tumor volume were measured 3 times per week after grouping for administration. The size of the mice tumor was measured using a vernier caliper each time, and the volume was calculated using the formula V = (L × W^2)/2, where L is the longest diameter of the tumor and W is the short diameter perpendicular to the longest diameter. On day 23 after grouping for administration, i.e., after the 4th administration, the experiment was terminated and all mice were euthanized.

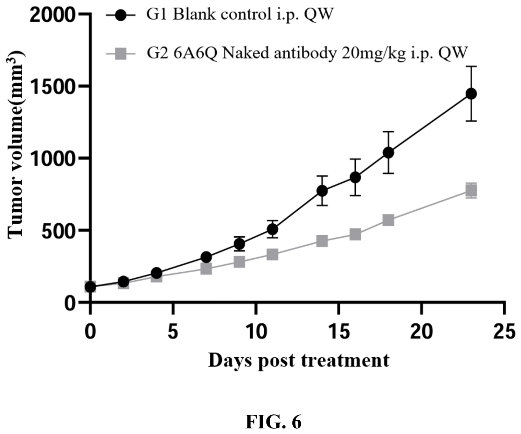

As shown in FIG. 6, based on data from the entire dosing cycle, the 6A6Q naked antibody group showed a significant inhibitory effect on tumor growth, with statistically significant differences compared to the blank control group (p<0.0001).

The final data show that the tumor volume of the blank control group was 1447.5±190.8mm³; the tumor volume in the 6A6Q naked antibody group was 776.3±52.4mm³. The results demonstrated that the TEM8 antibody drug 6A6Q naked antibody had stronger tumor inhibitory activity.

### Sequence listing

| **Sequence description** | **Sepcific sequence** | | **SEQ ID NO** |
|---|---|---|---|
| TEM8-3A3 | | | |
| TEM8-antibody heavy chain variable region (TEM8-3A3-VH) | QVQLVQSGPEVKKPGAPVKISCKTSGYIFDDYGIT WVRQAPGQGLEWLGWISTYNGKTSFAQKVQGRLT MTTDISTNTASMELRGLRSDDTALYYCARVFSRYI PFYFDSWGQGTLVTVSS | | 1 |
| TEM8-antibody light chain variable region (TEM8-3A3-VL) | DVVMTQSPDSLAVSLGERATINCKSSQSVLYSYNN KNYLAWYQQKPGQPPKLLIYWASTRESGVPDRFS GSGSGTDFTLTISSLQAEDVAVYYCQQYYSTPLTFG GGTKLEIK | | 2 |
| TEM8-antibody heavy chain variable region TEM8-3A3-VH-HCDR1 (IMGT) | GYIFDDYG | | 3 |
| TEM8-antibody heavy chain variable region TEM8-3A3-VH-HCDR2 (IMGT) | ISTYNGKT | | 4 |
| TEM8-antibody heavy chain variable region TEM8-3A3-VH-HCDR3 (IMGT) | ARVFSRYIPFYFDS | | 5 |
| TEM8-antibody light chain variable region TEM8-3A3-VL-LCDR1 (IMGT) | QSVLYSYNNKNY | | 6 |
| TEM8-antibody TEM8-3A3-VL-LCDR2 (IMGT) | WAS | | 7 |
| TEM8-antibodyTEM8-3A3-VL-LCDR3 (IMGT) | QQYYSTPLT | | 8 |
| TEM8-antibody heavy chain variable region TEM8-3A3-VH-HCDR1 ( Kabat) | DYGIT | | 9 |
| TEM8-antibody heavy chain variable region TEM8-3A3-VH-HCDR2 ( Kabat) | WISTYNGKTSFAQKVQG | | 10 |
| TEM8-antibody heavy chain variable region TEM8-3A3-VH-HCDR3 ( Kabat) | VFSRYIPFYFDS | | 11 |
| TEM8-antibody light chain variable region TEM8-3A3-VL-LCDR1 (Kabat) | KSSQSVLYSYNNKNYLA | | 12 |
| TEM8-antibody TEM8-3A3-VL-LCDR2 (Kabat) | WASTRES | | 13 |
| TEM8-antibody TEM8-3A3-VL-LCDR3 ( Kabat) | QQYYSTPLT | | 8 |

| TEM8-3B3 | | | |
|---|---|---|---|
| TEM8-antibody heavy chain variable region (TEM8-3B3-VH) | EVQLVQSGGGLVKPGGSLRLSCAASGFTFSSYSMN WVRQAPGKGLEWVSSISSSSSYIYYADSVKGRFTIS RDNAKNSLYLQMNSLRAEDTAVYYCARVRPPYG MDVWGQGTLVTVSS | | 14 |
| TEM8-antibody light chain variable region (TEM8-3B3-VL) | NIQMTQSPSSVSASVGDTVTISCRASQGISNWLGW YQQRPGEAPKLLIYAASHLQSGVPSRFSGSGSGTDF VLTITSLQPEDFATYYCQQSNSFPLAFGGGTKLEIK | | 15 |
| TEM8-antibody heavy chain variable region TEM8-3B3-VH-HCDR1 (IMGT) | GFTFSSYS | | 16 |
| TEM8-antibody heavy chain variable region TEM8-3B3-VH-HCDR2 (IMGT) | ISSSSSYI | | 17 |
| TEM8-antibody heavy chain variable region TEM8-3B3-VH-HCDR3 (IMGT) | ARVRPPYGMDV | | 18 |
| TEM8-antibody light chain variable region TEM8-3B3-VL-LCDR1 (IMGT) | QGISNW | | 19 |
| TEM8-antibody TEM8-3B3-VL-LCDR2 (IMGT) | AAS | | 20 |
| TEM8-antibody TEM8-3B3-VL-LCDR3 (IMGT) | QQSNSFPLA | | 21 |
| TEM8-antibody heavy chain variable region TEM8-3B3-VH-HCDR1 ( Kabat) | SYSMN | | 22 |
| TEM8-antibody heavy chain variable region TEM8-3B3-VH-HCDR2 ( Kabat) | SISSSSSYIYYADSVKG | | 23 |
| TEM8-antibody heavy chain variable region TEM8-3B3-VH-HCDR3 ( Kabat) | VRPPYGMDV | | 24 |
| TEM8-antibody light chain variable region TEM8-3B3-VL-LCDR1 (Kabat) | RASQGISNWLG | | 25 |
| TEM8-antibody TEM8-3B3-VL-LCDR2 (Kabat) | AASHLQS | | 26 |
| TEM8-antibody TEM8-3B3-VL-LCDR3 (Kabat) | QQSNSFPLA | | 21 |

| TEM8-6A6 | | | |
|---|---|---|---|
| TEM8-antibody heavy chain variable region (TEM8-6A6-VH) | EVQLVESGGGVVQPGGSLRLSCAASGFTFDDYAM HWVRQAPGKGLEWVSLISGDGGSTYYADSVKGRF TISRDNSKNSLYLQMNSLKASDTAMYYCARPRWG RYSSSWPFDYWGQGTLVTVSS | | 27 |
| TEM8-antibody light chain variable region (TEM8-6A6-VL) | QSALTQPASVSGSPGQSITISCTGTSSDVGTYNYVS WYQQHPGKAPKLMIYDVTDRPSGISNRFSGSKSGN TASLTISGLQAEDEGNYYCSSYTSNRSWVFGGGTK LTVL | | 28 |
| TEM8-antibody heavy chain variable region TEM8-6A6-VH-HCDR1 (IMGT) | GFTFDDYA | | 29 |
| TEM8-antibody heavy chain variable region TEM8-6A6-VH-HCDR2 (IMGT) | ISGDGGST | | 30 |
| TEM8-antibody heavy chain variable region TEM8-6A6-VH-HCDR3 (IMGT) | ARPRWGRYSSSWPFDY | | 31 |
| TEM8-antibody light chain variable region TEM8-6A6-VL-LCDR1 (IMGT) | SSDVGTYNY | | 32 |
| TEM8-antibody TEM8-6A6-VL-LCDR2 (IMGT) | DVT | | 33 |
| TEM8-antibody TEM8-6A6-VL-LCDR3 (IMGT) | SSYTSNRSWV | | 34 |
| TEM8-antibody heavy chain variable region TEM8-6A6-VH-HCDR1 (Kabat) | DYAMH | | 35 |
| TEM8-antibody heavy chain variable region TEM8-6A6-VH-HCDR2 (Kabat) | LISGDGGSTYYADSVKG | | 36 |
| TEM8-antibody heavy chain variable region TEM8-6A6-VH-HCDR3 (Kabat) | PRWGRYSSSWPFDY | | 37 |
| TEM8-antibody light chain variable region TEM8-6A6-VL-LCDR1 (Kabat) | TGTSSDVGTYNYVS | | 38 |
| TEM8-antibody TEM8-6A6-VL-LCDR2 (Kabat) | DVTDRPS | | 39 |
| TEM8-antibody TEM8-6A6-VL-LCDR3 (Kabat) | SSYTSNRSWV | | 34 |

| TEM8-6A6Q | | | |
|---|---|---|---|
| TEM8-antibody heavy chain variable region (TEM8-6A6Q-VH) | EVQLVESGGGVVQPGGSLRLSCAASGFTFDDYAM HWVRQAPGKGLEWVSLISGDGGSTYYADSVKGRF TISRDNSKNSLYLQMNSLKASDTAMYYCARPRWG RYSSSWPFDYWGQGTLVTVSS | | 27 |
| TEM8-antibody light chain variable region (TEM8-6A6Q-VL) | QSALTQPASVSGSPGQSITISCTGTSSDVGTYNYVS WYQQHPGKAPKLMIYDVTDRPSGISNRFSGSKSGN TASLTISGLQAEDEGNYYCSSYTSQRSWVFGGGTK LTVL | | 40 |
| TEM8-antibody heavy chain variable region TEM8-6A6Q-VH-HCDR1 (IMGT) | GFTFDDYA | | 29 |
| TEM8-antibody heavy chain variable region TEM8-6A6Q-VH-HCDR2 (IMGT) | ISGDGGST | | 30 |
| TEM8-antibody heavy chain variable region TEM8-6A6Q-VH-HCDR3 (IMGT) | ARPRWGRYSSSWPFDY | | 31 |
| TEM8-antibody light chain variable region TEM8-6A6Q-VL-LCDR1 (IMGT) | SSDVGTYNY | | 32 |
| TEM8-antibody TEM8-6A6Q-VL-LCDR2 (IMGT) | DVT | | 33 |
| TEM8-antibody TEM8-6A6Q-VL-LCDR3 (IMGT) | SSYTSQRSWV | | 41 |
| TEM8-antibody heavy chain variable region TEM8-6A6Q-VH-HCDR1 ( Kabat) | DYAMH | | 35 |
| TEM8-antibody heavy chain variable region TEM8-6A6Q-VH-HCDR2 ( Kabat) | LISGDGGSTYYADSVKG | | 36 |
| TEM8-antibody heavy chain variable region TEM8-6A6Q-VH-HCDR3 ( Kabat) | PRWGRYSSSWPFDY | | 37 |
| TEM8-antibody light chain variable region TEM8-6A6Q-VL-LCDR1 (Kabat) | TGTSSDVGTYNYVS | | 38 |
| TEM8-antibody TEM8-6A6Q-VL-LCDR2 (Kabat) | DVTDRPS | | 39 |
| TEM8-antibody TEM8-6A6Q-VL-LCDR3 ( Kabat) | SSYTSQRSWV | | 41 |

| TEM8-7E3 | | | |
|---|---|---|---|
| TEM8-antibody heavy chain variable region (TEM8-7E3-VH) | QVQLVESGGGLVNPGGSLRLSCAVSGFTSSGFSIN WVRQAPGKGLEWVSSISGIGDSIFYADSVKGRFTIS RDNAKNLVFLQMNSVKAEDTAVYYCVRDKWGW GTYSLFDYWGQGTLVTVSS | | 42 |
| TEM8-antibody light chain variable region (TEM8-7E3-VL) | QAVLTQPPSASGTPGQRVTISCSGSSSNIASNVVSW YQQLPGTAPKVLIYSNNRRPSGVPDRFSGSKSGAS ASLAISGLQSDDETHYYCAAWDDSLKGPVFGGGT QLTVL | | 43 |
| TEM8-antibody heavy chain variable region TEM8-7E3-VH-HCDR1 (IMGT) | GFTSSGFS | | 44 |
| TEM8-antibody heavy chain variable region TEM8-7E3-VH-HCDR2 (IMGT) | ISGIGDSI | | 45 |
| TEM8-antibody heavy chain variable | VRDKWGWGTYSLFDY | | 46 |
| region TEM8-7E3-VH-HCDR3 (IMGT) | | | |
| TEM8-antibody light chain variable region TEM8-7E3-VL-LCDR1 (IMGT) | SSNIASNV | | 47 |
| TEM8-antibody TEM8-7E3-VL-LCDR2 (IMGT) | SNN | | 48 |
| TEM8-antibody TEM8-7E3-VL-LCDR3 (IMGT) | AAWDDSLKGPV | | 49 |
| TEM8-antibody heavy chain variable region TEM8-7E3-VH-HCDR1 ( Kabat) | GFSIN | | 50 |
| TEM8-antibody heavy chain variable region TEM8-7E3-VH-HCDR2 ( Kabat) | SISGIGDSIFYADSVKG | | 51 |
| TEM8-antibody heavy chain variable region TEM8-7E3-VH-HCDR3 ( Kabat) | DKWGWGTYSLFDY | | 52 |
| TEM8-antibody light chain variable region TEM8-7E3-VL-LCDR1 (Kabat) | SGSSSNIASNVVS | | 53 |
| TEM8-antibody TEM8-7E3-VL-LCDR2 (Kabat) | SNNRRPS | | 54 |
| TEM8-antibody TEM8-7E3-VL-LCDR3 ( Kabat) | AAWDDSLKGPV | | 49 |
| TEM8-11B7 | | | |
| TEM8-antibody heavy chain variable region (TEM8-11B7-VH) | QVQLVQSGGGVVQPGRPLRLSCAASGFNLGSYAM SWVRQAPGKGLEWVSAISGSGGSTYYADSVKGRF TISRDNSKNTLYLQMNSLRAEDTAVYYCAKILGPG NLNYYYYYGMDVWGQGTTVTVSS | | 55 |
| TEM8-antibody light chain variable region (TEM8-11B7-VL) | SSELTQDPAVSVALGQTVRITCQGDSLRNSYASWY QQKPGQAPVLVIYGNNNRPSGIPDRVSGSRSGNTA SLTITGAQAEDEADYYCNSRDSSGNHVVFGGGTEL TVL | | 56 |
| TEM8-antibody heavy chain variable region TEM8-11B7-VH-HCDR1 (IMGT) | GFNLGSYA | | 57 |
| TEM8-antibody heavy chain variable region TEM8-11B7-VH-HCDR2 (IMGT) | ISGSGGST | | 58 |
| TEM8-antibody heavy chain variable region TEM8-11B7-VH-HCDR3 (IMGT) | AKILGPGNLNYYYYYGMDV | | 59 |
| TEM8-antibody light chain variable region TEM8-11B7-VL-LCDR1 (IMGT) | SLRNSY | | 60 |
| TEM8-antibody TEM8-11B7-VL-LCDR2 (IMGT) | GNN | | 61 |
| TEM8-antibody TEM8-1IB7-VL-LCDR3 (IMGT) | NSRDSSGNHVV | | 62 |
| TEM8-antibody heavy chain variable region TEM8-11B7-VH-HCDR1 ( Kabat) | | SYAMS | 63 |
| TEM8-antibody heavy chain variable region TEM8-11B7-VH-HCDR2 ( Kabat) | | AISGSGGSTYYADSVKG | 64 |
| TEM8-antibody heavy chain variable region TEM8-11B7-VH-HCDR3 ( Kabat) | | ILGPGNLNYYYYYGMDV | 65 |
| TEM8-antibody light chain variable region TEM8-11B7-VL-LCDR1 (Kabat) | | QGDSLRNSYAS | 66 |
| TEM8-antibody TEM8-1IB7-VL-LCDR2 (Kabat) | | GNNNRPS | 67 |
| TEM8-antibody TEM8-1IB7-VL-LCDR3 ( Kabat) | | NSRDSSGNHVV | 62 |

| Other sequence | | | |
|---|---|---|---|
| Human TEM8 | EDGGPACYGGFDLYFILDKSGSVLHHWNEIYYFVEQLAHKFIS PQLRMSFIVFSTRGTTLMKLTEDREQIRQGLEELQKVLPGGDT YMHEGFERASEQIYYENRQGYRTASVIIALTDGELHEDLFFYS EREANRSRDLGAIVYCVGVKDFNETQLARIADSKDHVFPVND GFQALQGIIHSILKKSCIEILAAEPSTICAGESFQVVVRGNGFRH ARNVDRVLCSFKINDSVTLNEKPFSVEDTYLLCPAPILKEVGM KAALQVSMNDGLSFISSSVIITTTHCSDGSILAIALLILFLLLAL ALLWWFWPLCCTVIIKEVPPPPAEESEEEDDDGLPKKKWPTV DASYYGGRGVGGIKRMEVRWGEKGSTEEGAKLEKAKNARV KMPEQEYEFPEPRNLNNNMRRPSSPRKWYSPIKGKLDALWVL LRKGYDRVSVMRPQPGDTGRCINFTRVKNNQPAKYPLNNAYH TSSPPPAPIYTPPPPAPHCPPPPPSAPTPPIPSPPSTLPPPPQAPPPN RAPPPSRPPPRPSV | | 68 |
| Mouse TEM8 | EDGGPACYGGFDLYFILDKSGSVLHHWNEIYYFVEQLAHRFIS PQLRMSFIVFSTRGTTLMKLTEDREQIRQGLEELQKVLPGGDT YMHEGFERASEQIYYENSQGYRTASVIIALTDGELHEDLFFYSE REANRSRDLGAIVYCVGVKDFNETQLARIADSKDHVFPVNDG FQALQGIIHSILKKSCIEILAAEPSTICAGESFQVVVRGNGFRHA RNVDRVLCSFKINDSVTLNEKPFAVEDTYLLCPAPILKEVGMK AALQVSMNDGLSFISSSVIITTTHCSDGSILAIALLVLFLLLALA LLWWFWPLCCTVIIKEVPPPPVEESEEEDDDGLPKKKWPTVD ASYYGGRGVGGIKRMEVRWGEKGSTEEGAKLEKAKNARVK MPEQEYEFPEPRNLNNNMRRPSSPRKWYSPIKGKLDALWVLL RKGYDRVSVMRPQPGDTGRCINFTRVKNSQPAKYPLNNTYHP SSPPPAPIYTPPPPAPHCPPPAPSAPTPPIPSPPSTLPPPPQAPPPNR APPPSRPPPRPSV | | 69 |
| Dog TEM8 | EDAGAACYGGFDLYFILDKSGSVLHHWNEIYYFVEQLAHKFI SPQLRMSFIVFSTRGTTLMKLTEDREQIRQGLEELQKVLPGGD TYMHEGFERASEQIYYENSQGYRTASVIIALTDGELHEDLFFYS EREANRSRDLGAIVYCVGVKDFNETQLARIADSKDHVFPVND GFQALQGIIHSILKKSCIEILAAEPSTICAGESFQVVVRGNGFRH ARNVDRVLCSFKINESVTLNEKPFTVEDTYLLCPAPILKEVGM KAALQVSMNDGLSFISSSVIITTTRCSDGSILAIALLILFLLLAL ALLWWFWPLCCTVIIKEVPPPPAEESEEEDDDGLPKKKWPTV | | 70 |
| | DASYYGGRGVGGIKRMEVRWGEKGSTEEGAKLEKAKNARV KMPEQEYEFPEPRNLNNNLRRPSSPRKWYSPIKGKLDALWVL LRKGYDRVSVMRPQPGDTGRCINFTRVKNNQPAKYPLNNAYH TSSPPPAPIYTPPPPAPHCPPPPPSAPTPPIPSPPSTLPPPPHAPPPS RAPPPSRPPPRPSV | | |
| IgG heavy chain constant region | ASTKGPsVFPLAPSSKSTSGGTAALGCLVKDYFPEPVTVSWNS GALTSGVHTFPAVLQSSGLYSLSSVVTVPSSSLGTQTYICNVNH KPSNTKVDKKVEPKSCDKTHTCPPCPAPEAAGGPSVFLFPPKP KDTLMISRTPEVTCVVVDVSHEDPEVKFNWYVDGVEVHNAK TKPREEQYNSTYRVVSVLTVLHQDWLNGKEYKCKVSNKALG APIEKTISKAKGQPREPQVYTLPPSRDELTKNQVSLTCLVKGFY PSDIAVEWESNGQPENNYKTTPPVLDSDGSFFLYSKLTVDKSR WQQGNVFSCSVMHEALHNHYTQKSLSLSPGK | | 71 |
| IgG light chain constant region (Kappa) | | | 72 |
| IgG light chain constant region (Lambda) | GQPKAAPSVTLFPPSSEELQANKATLVCLISDFYPGAVTVAWK ADSSPVKAGVETTTPSKQSNNKYAASSYLSLTPEQWKSHRSYS CQVTHEGSTVEKTVAPTECS | | 73 |
| L2 antibody heavy chain sequence | QVQLKESGPALVKPTQTLTLTCTFSGFSLSTSGGGVSWIRQPPG KALEWLAHIYSNDDKSYSTSLKTRLTISKDTSKNQVVLTMTN MDPVDTATYYCARGGYFLDYWGQGTLVTVSSASTKGPSVFPL APSSKSTSGGTAALGCLVKDYFPEPVTVSWNSGALTSGVHTFP AVLQSSGLYSLSSVVTVPSSSLGTQTYICNVNHKPSNTKVDKK VEPKSCDKTHTCPPCPAPELLGGPSVFLFPPKPKDTLMISRTPE VTCVVVDVSHEDPEVKFNWYVDGVEVHNAKTKPREEQYNS TYRVVSVLTVLHQDWLNGKEYKCKVSNKALPAPIEKTISKAK GQPREPQVYTLPPSRDELTKNQVSLTCLVKGFYPSDIAVEWES NGQPENNYKTTPPVLDSDGSFFLYSKLTVDKSRWQQGNVFSC SVMHEALHNHYTQKSLSLSPGK | | 74 |
| L2 antibody light chain sequence | DIELTQPPSVSVAPGQTARISCSGDNIGGIYVHWYQQKPGQAP VLVIYADSKRPSGIPERFSGSNSGNTATLTISGTQAEDEADYYC QSYDITSLVFGGGTKLTVLGQPKAAPSVTLFPPSSEELQANKAT LVCLISDFYPGAVTVAWKADSSPVKAGVETTTPSKQSNNKYA ASSYLSLTPEQWKSHRSYSCQVTHEGSTVEKTVAPTECS | | 75 |

## Claims

1. An antibody specifically binding to TEM8 or an antigen-binding fragment thereof, wherein the antibody comprises:
1) 3 complementarity-determining regions HCDR1, HCDR2, and HCDR3 contained in VH as set forth in SEQ ID NO: 1, and 3 complementarity-determining regions LCDR1, LCDR2, and LCDR3 contained in VL as set forth in SEQ ID NO: 2;
2) 3 complementarity-determining regions HCDR1, HCDR2, and HCDR3 contained in VH as set forth in SEQ ID NO: 14, and 3 complementarity-determining regions LCDR1, LCDR2, and LCDR3 contained in VL as set forth in SEQ ID NO: 15;
3) 3 complementarity-determining regions HCDR1, HCDR2, and HCDR3 contained in VH as set forth in SEQ ID NO: 27, and 3 complementarity-determining regions LCDR1, LCDR2, and LCDR3 contained in VL as set forth in SEQ ID NO: 28 or 40;
4) 3 complementarity-determining regions HCDR1, HCDR2, and HCDR3 contained in VH as set forth in SEQ ID NO: 42, and 3 complementarity-determining regions LCDR1, LCDR2, and LCDR3 contained in VL as set forth in SEQ ID NO: 43; or
5) 3 complementarity-determining regions HCDR1, HCDR2, and HCDR3 contained in VH as set forth in SEQ ID NO: 55, and 3 complementarity-determining regions LCDR1, LCDR2, and LCDR3 contained in VL as set forth in SEQ ID NO: 56;
preferably, wherein the HCDR and the LCDR are determined according to the IMGT or Kabat scheme.

2. An antibody specifically binding to TEM8 or an antigen-binding fragment thereof, wherein the antibody comprises: 3 complementarity-determining regions HCDR1, HCDR2 and HCDR3 of the heavy chain variable region and 3 complementarity-determining regions LCDR1, LCDR2 and LCDR3 of the light chain variable region, wherein the HCDR1, HCDR2, HCDR3, LCDR1, LCDR2, and LCDR3 are based on the IMGT scheme, comprising or consisting of, respectively, the amino acid sequence as set forth in SEQ ID NOs in the table below:
| HCDR1 (IMGT) comprising or consisting of the SEQ ID NOs below: | HCDR2 (IMGT) comprising or consisting of the SEQ ID NOs below: | HCDR3 (IMGT) comprising or consisting of the SEQ ID NOs below: | LCDR1 (IMGT) comprising or consisting of the SEQ ID NOs below: | LCDR2 (IMGT) comprising or consisting of the SEQ ID NOs below: | LCDR3 (IMGT) comprising or consisting of the SEQ ID NOs below: |
|---|---|---|---|---|---|
| 3 | 4 | 5 | 6 | 7 | 8 |
| 16 | 17 | 18 | 19 | 20 | 21 |
| 29 | 30 | 31 | 32 | 33 | 34 |
| 29 | 30 | 31 | 32 | 33 | 41 |
| 44 | 45 | 46 | 47 | 48 | 49 |
| 57 | 58 | 59 | 60 | 61 | 62 |
or wherein the HCDR1, HCDR2, HCDR3, LCDR1, LCDR2, and LCDR3 are based on the Kabat scheme, comprising or consisting of, respectively, the amino acid sequence as set forth in SEQ ID NOs in the table below:
| HCDR1 (Kabat) comprising or consisting of the SEQ ID NOs below: | HCDR2 (Kabat) comprising or consisting of the SEQ ID NOs below: | HCDR3 (Kabat) comprising or consisting of the SEQ ID NOs below: | LCDR1 (Kabat) comprising or consisting of the SEQ ID NOs below: | LCDR2 (Kabat) comprising or consisting of the SEQ ID NOs below: | LCDR3 (Kabat) comprising or consisting of the SEQ ID NOs below: |
|---|---|---|---|---|---|
| 9 | 10 | 11 | 12 | 13 | 8 |
| 22 | 23 | 24 | 25 | 26 | 21 |
| 35 | 36 | 37 | 38 | 39 | 34 |
| 35 | 36 | 37 | 38 | 39 | 41 |
| 50 | 51 | 52 | 53 | 54 | 49 |
| 63 | 64 | 65 | 66 | 67 | 62 |

3. The TEM8-binding antibody or antigen-binding fragment thereof according to claim 1 or 2, wherein the antibody comprises a heavy chain variable region, wherein the heavy chain variable region comprises or consists an amino acid sequence having at least 90%, 91%, 92%, 93%, 94%, 95%, 96%, 97%, 98%, or 99% identity to the amino acid sequence selected from the group consisting of SEQ ID NOs: 1, 14, 27, 42, or 55; or the heavy chain variable region comprises or consists of the amino acid sequence selected from the group consisting of SEQ ID NOs: 1, 14, 27, 42, or 55; and/or
the antibody comprises a light chain variable region, wherein the light chain variable region comprises or consists an amino acid sequence having at least 90%, 91%, 92%, 93%, 94%, 95%, 96%, 97%, 98%, or 99% identity to the amino acid sequence selected from the group consisting of SEQ ID NOs: 2, 15, 28, 40, 43, or 56; or the light chain variable region comprises or consists of the amino acid sequence selected from the group consisting of SEQ ID NOs: 2, 15, 28, 40, 43, or 56.

4. An anti-TEM8 antibody or antigen-binding fragment thereof, wherein the antibody comprises a heavy chain variable region and a light chain variable region, wherein
(i) the heavy chain variable region comprises an amino acid sequence having at least 90%, 91%, 92%, 93%, 94%, 95%, 96%, 97%, 98%, or 99% identity to the amino acid sequence as set forth in SEQ ID NO: 1, and the light chain variable region comprises an amino acid sequence having at least 90%, 91%, 92%, 93%, 94%, 95%, 96%, 97%, 98%, or 99% identity to the amino acid sequence as set forth in SEQ ID NO: 2;
(ii) the heavy chain variable region comprises an amino acid sequence having at least 90%, 91%, 92%, 93%, 94%, 95%, 96%, 97%, 98%, or 99% identity to the amino acid sequence as set forth in SEQ ID NO: 14, and the light chain variable region comprises an amino acid sequence having at least 90%, 91%, 92%, 93%, 94%, 95%, 96%, 97%, 98%, or 99% identity to the amino acid sequence as set forth in SEQ ID NO: 15;
(iii) the heavy chain variable region comprises an amino acid sequence having at least 90%, 91%, 92%, 93%, 94%, 95%, 96%, 97%, 98%, or 99% identity to the amino acid sequence as set forth in SEQ ID NO: 27, and the light chain variable region comprises an amino acid sequence having at least 90%, 91%, 92%, 93%, 94%, 95%, 96%, 97%, 98%, or 99% identity to the amino acid sequence as set forth in SEQ ID NO: 28 or 40;
(iv) the heavy chain variable region comprises an amino acid sequence having at least 90%, 91%, 92%, 93%, 94%, 95%, 96%, 97%, 98%, or 99% identity to the amino acid sequence as set forth in SEQ ID NO: 42, and the light chain variable region comprises an amino acid sequence having at least 90%, 91%, 92%, 93%, 94%, 95%, 96%, 97%, 98%, or 99% identity to the amino acid sequence as set forth in SEQ ID NO: 43;
(v) the heavy chain variable region comprises an amino acid sequence having at least 90%, 91%, 92%, 93%, 94%, 95%, 96%, 97%, 98%, or 99% identity to the amino acid sequence as set forth in SEQ ID NO: 55, and the light chain variable region comprises an amino acid sequence having at least 90%, 91%, 92%, 93%, 94%, 95%, 96%, 97%, 98%, or 99% identity to the amino acid sequence as set forth in SEQ ID NO: 56.

5. The TEM8-binding antibody or antigen-binding fragment thereof according to claim 1 or 2, wherein the antibody comprises a heavy chain variable region and a light chain variable region, wherein the heavy chain variable region and the light chain variable region comprise or consist of, respectively, the amino acid sequences as set forth in SEQ ID NOs in the table below:
| Heavy chain variable region | Light chain variable region |
|---|---|
| SEQ ID NO: 1 | SEQ ID NO: 2 |
| SEQ ID NO: 14 | SEQ ID NO: 15 |
| SEQ ID NO: 27 | SEQ ID NO: 28 |
| SEQ ID NO: 27 | SEQ ID NO: 40 |
| SEQ ID NO: 42 | SEQ ID NO: 43 |
| SEQ ID NO: 55 | SEQ ID NO: 56 |

6. The TEM8-binding antibody or antigen-binding fragment thereof according to any one of claims 1-5, wherein the antibody comprises a heavy chain constant region, for example, a heavy chain constant region of IgG1, IgG2, IgG3, or IgG4, e.g., a heavy chain constant region of IgG1, which
(i) comprises an amino acid sequence having at least 85%, 90%, 91%, 92%, 93%, 94%, 95%, 96%, 97%, 98%, or 99% identity to the amino acid sequence selected from the group consisting of SEQ ID NO: 71; or
(ii) comprises or consists of the amino acid sequence selected from the group consisting of SEQ ID NO: 71.

7. The TEM8-binding antibody or antigen-binding fragment thereof according to any one of claims 1-6, wherein the antibody comprises a light chain constant region, for example, a lambda or kappa light chain constant region, e.g., the light chain constant region
(i) comprises or consists of an amino acid sequence having at least 85%, 90%, 91%, 92%, 93%, 94%, 95%, 96%, 97%, 98%, or 99% identity to the amino acid sequence selected from the group consisting of SEQ ID NO: 72 or 73; or
(ii) comprises or consists of an amino acid sequence selected from the group consisting of SEQ ID NO: 72 or 73.

8. The TEM8-binding antibody or antigen-binding fragment thereof according to any one of claims 1-7, wherein the antibody comprises a heavy chain constant region and a light chain constant region, wherein
the heavy chain constant region comprises or consists of the amino acid sequence as set forth in SEQ ID NO: 71, and the light chain constant region comprises or consists of the amino acid sequence as set forth in SEQ ID NO: 72 or 73.

9. The TEM8-binding antibody or antigen-binding fragment thereof according to any one of claims 1-8, wherein the antibody comprises a heavy chain and a light chain, wherein the heavy chain comprises a heavy chain variable region and a heavy chain constant region, and the light chain comprises a light chain variable region and a light chain constant region, wherein
(i). the heavy chain variable region comprises or consists of the amino acid sequence as set forth in SEQ ID NO: 1, the light chain variable region comprises or consists of the amino acid sequence as set forth in SEQ ID NO: 2, the heavy chain constant region comprises or consists of the amino acid sequence as set forth in SEQ ID NO: 71, and the light chain constant region comprises or consists of the amino acid sequence as set forth in SEQ ID NO: 72;
(ii). the heavy chain variable region comprises or consists of the amino acid sequence as set forth in SEQ ID NO: 14, the light chain variable region comprises or consists of the amino acid sequence as set forth in SEQ ID NO: 15, the heavy chain constant region comprises or consists of the amino acid sequence as set forth in SEQ ID NO: 71, and the light chain constant region comprises or consists of the amino acid sequence as set forth in SEQ ID NO: 72;
(iii). the heavy chain variable region comprises or consists of the amino acid sequence as set forth in SEQ ID NO: 27, the light chain variable region comprises or consists of the amino acid sequence as set forth in SEQ ID NO: 28 or 40, the heavy chain constant region comprises or consists of the amino acid sequence as set forth in SEQ ID NO: 71, and the light chain constant region comprises or consists of the amino acid sequence as set forth in SEQ ID NO: 73;
(iv). the heavy chain variable region comprises or consists of the amino acid sequence as set forth in SEQ ID NO: 42, the light chain variable region comprises or consists of the amino acid sequence as set forth in SEQ ID NO: 43, the heavy chain constant region comprises or consists of the amino acid sequence as set forth in SEQ ID NO: 71, and the light chain constant region comprises or consists of the amino acid sequence as set forth in SEQ ID NO: 73; or
(v). the heavy chain variable region comprises or consists of the amino acid sequence as set forth in SEQ ID NO: 55, the light chain variable region comprises or consists of the amino acid sequence as set forth in SEQ ID NO: 56, the heavy chain constant region comprises or consists of the amino acid sequence as set forth in SEQ ID NO: 71, and the light chain constant region comprises or consists of the amino acid sequence as set forth in SEQ ID NO: 73.

10. The TEM8-binding antibody or antigen-binding fragment thereof according to any one of claims 1-8, having one or more of the following properties:
(1) being capable of binding with high affinity to TEM8, e.g., to TEM8 of human, cynomolgus monkey, rat, or mouse;
(2) being capable of binding with high affinity to TEM8 expressed on the cell membrane surface, e.g., to TEM8 of human, cynomolgus monkey, rat, or mouse;
(3) being capable of inducing endocytosis of the antibody or fragment thereof or a molecule comprising the antibody or fragment thereof (e.g., an ADC) by a TEM8-positive cell (e.g., a TEM8-overexpressing CHO cell);
(4) being capable of targeting and killing a TEM8-positive cell, such as a TEM8-positive tumor cell;
(5) being capable of treating a tumor, such as a cancer;
(6) being suitable for constructing an antibody-drug conjugate (ADC) for targeting and killing TEM8-positive cells, such as TEM8-positive tumor cells, or for the treatment of tumors such as cancer;
(7) inhibiting binding of any of the antibodies listed in claim 9 to TEM8;
(8) binding to the same or an overlapping epitope as any one of the antibodies of claim 9; or
(9) competing for binding to TEM8 with any one of the antibodies of claim 9.

11. The antibody or antigen-binding fragment thereof according to any one of claims 1-10, wherein the antibody is a monoclonal antibody.

12. The antibody or antigen-binding fragment thereof according to any one of claims 1-11, wherein the antibody is a human antibody.

13. The antibody or antigen-binding fragment thereof according to any one of claims 1-12, wherein the antigen-binding fragment is an antibody fragment selected from the group consisting of Fab, Fab', Fab'-SH, Fv, a single chain antibody (e.g., scfv), a (Fab')2 fragment, a single domain antibody, a diabody, or a linear antibody.

14. The antibody or antigen-binding fragment thereof according to claim 13, wherein the single-chain antibody is scFv.

15. The antibody or antigen-binding fragment thereof according to any one of claims 1-14, wherein the antibody is a bispecific or multispecific antibody.

16. An isolated nucleic acid encoding the anti-TEM8 antibody or antigen-binding fragment thereof of any one of claims 1-15.

17. An expression vector comprising the nucleic acid according to claim 16.

18. The expression vector according to claim 17, wherein the expression vector is a pCDNA vector, e.g., a pCDNA3.4 vector.

19. A host cell comprising the nucleic acid of claim 16 or the expression vector of claim 17 or 18.

20. The host cell according to claim 19, wherein the host cell is prokaryotic or eukaryotic.

21. The host cell according to claim 20, wherein the host cell is selected from the group consisting of *Escherichia coli* cells, yeast cells, mammalian cells, or other cells suitable for preparing an antibody or antigen-binding fragment thereof.

22. The host cell according to claim 21, wherein the host cell is a 293 cell or a CHO cell.

23. A method of preparing an anti-TEM8 antibody or antigen-binding fragment thereof, comprising culturing the host cell of any one of claims 19-22 under conditions suitable for expression of the nucleic acid encoding the anti-TEM8 antibody or antigen-binding fragment thereof of any one of claims 1-15, optionally isolating the antibody or antigen-binding fragment thereof, optionally the method further comprising recovering the anti-TEM8 antibody or antigen-binding fragment thereof from the host cell.

24. An antibody-drug conjugate comprising the antibody or antigen-binding fragment thereof of any one of claims 1-15 and additional therapeutic agents.

25. The antibody-drug conjugate according to claim 24, wherein the additional therapeutic agent is a cytotoxic agent, e.g., an MMAE, optionally the MMAE conjugates to an antibody or antigen-binding fragment thereof via a Vc linker, e.g., the antibody or antigen-binding fragment thereof is linked to VcMMAE to form an antibody-drug conjugate.

26. A pharmaceutical composition comprising the antibody or antigen-binding fragment thereof of any one of claims 1-15 or the antibody-drug conjugate of claim 24 or 25, and optionally a pharmaceutically acceptable auxiliary material.

27. A pharmaceutical composition comprising the antibody or antigen-binding fragment thereof of any one of claims 1-15 or the antibody-drug conjugate of claim 24 or 25, and additional therapeutic agents.

28. A pharmaceutical combination comprising the antibody or antigen-binding fragment thereof according to any one of claims 1-15 or the antibody-drug conjugate of claim 24 or 25, and additional therapeutic agents.

29. The pharmaceutical composition of claim 27 or the pharmaceutical combination of claim 28, wherein the additional therapeutic agents are selected from the group consisting of chemotherapeutic agents, cytokines, cytotoxic agents, other antibodies, small molecule drugs, or immunomodulators (e.g., immunosuppressants).

30. Use of an effective amount of the anti-TEM8 antibody or antigen-binding fragment thereof of any one of claims 1-15, or the antibody-drug conjugate of claim 24 or 25, or the pharmaceutical composition of claim 26, in the preparation of a medicament for preventing or treating a tumor in a subject or individual.

31. The use according to claim 30, wherein the tumor is a solid tumor, e.g., the tumor is a cancer, e.g., the cancer is selected from the group consisting of colon cancer, rectal cancer, or colorectal cancer.

32. The use according to claim 30 or 31, further comprising administering to the subject one or more additional therapies in combination.

33. The use according to claim 32, wherein the therapy comprises treatment modalities and/or additional therapeutic agents.

34. The use according to claim 33, wherein the additional therapeutic agents are selected from the group consisting of chemotherapeutic agents, cytokines, other antibodies, cytotoxic agents, vaccines, small molecule drugs, or immunomodulators (e.g., immunosuppressants), and/or the treatment modalities comprise surgical treatments and/or radiation therapies.

35. A method of detecting TEM8 in a sample comprising
(a) contacting the sample with the anti-TEM8 antibody or antigen-binding fragment thereof of any one of claims 1-15; and
(b) detecting the formation of a complex between an anti-TEM8 antibody or antigen-binding fragment thereof and TEM8; optionally, the anti-TEM8 antibody is detectably labeled.
